# EUROPEAN PATENT APPLICATION

(11) **EP 4 481 020 A2**
(43) Date of publication of application: **25.12.2024**
(21) Application number: 24193019.7
(22) Date of filing: 14.06.2017
(51) Int. Cl.: C10M 133/14

(54) **POLYISOBUTYLENE-SUBSTITUTED PHENOL, DERIVATIVES THEREOF, AND LUBRICATING COMPOSITIONS CONTAINING THE POLYISOBUTYLENESUBSTITUTED PHENOL AND ITS DERIVATIVES**

(30) Priority: 17.06.2016 US 201662351483 P; 09.06.2017 US 201762517281 P
(62) Divisional of application: 17733273.1
(71) Applicant: The Lubrizol Corporation, Wickliffe, OH 44092-2298 (US)
(72) Inventor: SAMPLER, Edward P., Belper, DE56 1QN (GB); WALKER, Gary M., Belper, DE56 1QN (GB); COOK, Stephen J., Belper, DE56 1QN (GB); KILSBY, Samuel M., Belper, DE56 1QN (GB)
(74) Representative: D Young & Co LLP

(57) **Abstract**

A polyisobutylene-substituted hydroxyaromatic compound and derivatives thereof which are suitable for use as additives in lubricating compositions. The polyisobutylene-substituted hydroxyaromatic compound may include the reaction product of a hydroxyaromatic compound with a polyisobutylene having a number average molecular weight of 150 to 800.

## Description

### BACKGROUND

The invention relates generally to a polyisobutylene-substituted phenol and derivatives thereof which may be used in lubricating compositions (lubricants).

Derivatives of phenols, such as phenol-based detergents are known. These include, for example, phenates based on phenolic monomers linked with sulfur bridges or alkylene bridges such as methylene linkages derived from formaldehyde. The phenolic monomers themselves are typically substituted with an aliphatic hydrocarbyl group to provide a measure of oil solubility. The hydrocarbyl groups may be alkyl groups, such as dodecylphenol (or propylene tetramer-substituted phenol). Basic sulfurized polyvalent metal phenates are described, for example, in U.S. Pat. No. 2,680,096, to Walker et al., issued June 1, 1954; and U.S. Pat. No. 3,372,116, to Meinhardt, issued March 6, 1968.

Recently, however, certain alkylphenols and products prepared from them have come under increased scrutiny due to their classification as potentially harmful materials. In particular, alkylphenol detergents which are based on oligomers of C₁₂ alkyl phenols may contain residual monomeric C₁₂ alkyl phenol species. These detergents include those derived from para-dodecyl phenol. There is interest, therefore, in developing alkyl-substituted phenate detergents, for uses in lubricants, fuels, and as industrial additives, which reduce or eliminate the need for dodecylphenol-based compounds.

There have been several efforts to prepare phenol derived detergents that do not contain Cₙ alkyl phenols. U.S. Pat. No. 7,435,709, to Stonebraker, et al., issued October 14, 2008, discloses a linear alkylphenol-derived detergent which is a salt of a reaction product of an olefin having at least 10 carbon atoms and a hydroxyaromatic compound. Greater than 90 mole % of the olefin is a linear C₂₀-C₃₀ n-alpha olefin, less than 10 mole % of the olefin is a linear olefin of less than 20 carbon atoms, and less than 5 mole %of the olefin is a branched chain olefin of 18 carbons or less.

U.S. Pub. No. 2011/0190185, to Sinquin, et al, published August 4, 2011, discloses an overbased salt of an oligomerized alkylhydroxyaromatic compound. The alkyl group is derived from an olefin mixture comprising propylene oligomers having an initial boiling point of at least 195 °C and a final boiling point of greater than 325 °C. The propylene oligomers may contain a distribution of carbon atoms that include at least 50 wt. % C₁₄ to C₂₀ carbon atoms.

U.S. Pub. No. 2011/0124539, to Sinquin, et al, published May 26, 2011, discloses an overbased, sulfurized salt of an alkylated hydroxyaromatic compound. The alkyl substituent is a residue of at least one isomerized olefin having from 15 to 99 wt. % branching. The hydroxyaromatic compound may be a phenol, cresol, xylenol, or mixture thereof.

U.S. Pub. No. 2011/0118160, to Campbell, et al., published May 19, 2011, discloses an alkylated hydroxyaromatic compound prepared by reacting a hydroxyaromatic compound with at least one branched olefinic propylene oligomer having from 20 to 80 carbon atoms. Suitable hydroxyaromatic compounds include phenol, catechol, resorcinol, hydroquinone, pyrogallol, cresol, and the like.

U.S. Pub. No. 2010/0029529, to Campbell, et al., published February 4, 2010, discloses an overbased salt of an oligomerized alkylhydroxyaromatic compound. The alkyl group is derived from an olefin mixture comprising propylene oligomers having an initial boing point of at least 195°C and a final boiling point of no more than 325 °C.

U.S. Pub. No. 2008/0269351, to Campbell, et al., published October 30, 2008, discloses an alkylated hydroxyaromatic compound prepared by reacting a hydroxyaromatic compound with a branched olefinic oligomer having from 20 to 80 carbon atoms.

U.S. Pat. No. 6,310,009, to Carrick, et al., issued October 30, 2001, discloses bridged phenolic compounds, each phenol group being substituted with an alkyl group of 1 to 60 carbon atoms, e.g., 9 to 18 carbon atoms.

There remains a need for a phenolic material with appropriate oil solubility, viscosity performance, and detergency (characteristic of moderate chain length alkyl groups) but free from or substantially free from C₁₂ alkyl phenol moieties.

### BRIEF DESCRIPTION

The present invention includes a polyisobutylene-substituted phenol having the formula: 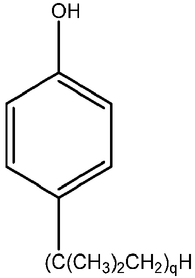 where q is, for example, from 4 to 10, or up to 7 or at least 6, and comprises the reaction product of a phenol with a polyisobutylene having a number average molecular weight of 175 to 500.

The present invention also comprises derivatives of this polyisobutylene-substituted phenol, including but not limited to, phenate detergents, Mannich detergents, oil-soluble zinc salts, polyethers or polyetheramines, methylene coupled hydrocarbyl phenols, or hydrocarbyl substituted amino phenols.

The present invention also comprises a lubricating composition comprising an oil of lubricating viscosity and the polyisobutylene-substituted phenol described herein or derivatives thereof as described herein.

### DETAILED DESCRIPTION

The present invention relates to a polyisobutylene-substituted hydroxyaromatic compound, such as a phenol, derivatives thereof, and the use of the polyisobutylene-substituted hydroxyaromatic compound or the derivatives in a lubricating composition.

Unless otherwise stated, molecular weight herein is reported as number average molecular weight, in Daltons, and is measured by Gel Permeation Chromatography (GPC) using a polyisobutylene standard.

In one embodiment, the polyisobutylene-substituted phenol compound has the formula: 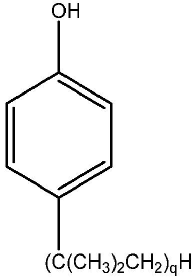 where q is, for example, from 4 to 10, or up to 7, or at least 6 and comprises the reaction product of a phenol with a polyisobutylene having a number average molecular weight of 175 to 500. In one embodiment, q may be 4, 5, 6, 7, 8, 9, 10, 11, 12, or mixtures of one or more of these. In one particular embodiment, the polyisobutylene-substituted phenol compound comprises the reaction product of a hydroxyaromatic compound, such as a phenol, with a polyisobutylene having a number average molecular weight of 300 to 400.

Suitable hydroxyaromatic compounds include phenol, catechol, resorcinol, hydroquinone, pyrogallol, cresol, and the like. While phenols are used as an example for this invention, it should be understood that other hydroxyaromatic compounds are within the scope of the present invention.

The term "substantially free" as it refers to the exemplary compounds described herein, means that less than 0.01 mol. %, or less than 0.001 mol. %, or less than 0.0001 mol. % of the polyisobutylene-substituted hydroxyaromatic compound in the lubricating composition is substituted with one or more C₁₂ alkyl groups. In one embodiment, the lubricating compound contains less than 0.5 wt. %, or less than 0.1 wt. %, or less than 0.01 wt. %, or less than 0.001 wt. %, of C₁₂ alkyl hydroxyaromatic compounds.

### Formation of the Polvisobutvlene-Substituted Phenol Compound

The polyisobutylene-substituted phenol intermediate compound of the present invention may be formed by reaction of a phenol with a poly(branched alkene). Substitution occurs primarily at the para position, although minor amounts of *ortho-* and/or *meta-* substitution may occur. The ratio of poly(branched alkene) to phenol may be from 1:1 to 1:6, such as at least 1:2. An excess of phenol helps to ensure primarily mono-substitution of the poly(branched alkene).

In one embodiment, the poly(branched alkene) may be a polysobutylene. For example, the polyisobutylene may be a highly reactive polyisobutylene, which differs from the polyisobutylenes having low reactivity through the content of terminally arranged ethylenic double bonds. Suitable highly reactive polyisobutylenes are, for example, polyisobutylenes which have more than 70, in particular more than 80, especially more than 85, mol %, based on the polyisobutylene macromolecules, of vinylidene double bonds. Particularly preferred polyisobutylenes are those which have uniform polymer backbones. In particular, those polymers which are composed of at least 85, preferably at least 90, particularly preferably at least 95, % by weight of isobutene units have uniform polymer backbones. In one useful embodiment, a highly reactive polyisobutylene used for the alkylation of a phenol as described herein will have a number average molecular weight of at least about 150, or at least about 200, or at least about 300, or up to 800 or up to 600, or up to 500, or up to 400, or even up to 360. In one embodiment, the polyisobutylene has a number average molecular weight of 150 to 500, or even 200 to 500, or even 300 to 400. In some embodiments, useful polyisobutylenes may have a polydispersity of greater than 1.5, for example 1.5 to 3.0. Polydispersity is understood as meaning the quotient of weight average molecular weight M_{w} and number average molecular weight M_{N}.

In another embodiment, the polyisobutylene useful in this invention is made in the absence of a chain transfer agent. CTAs are known in literature. For example, J. P. Kennedy et al, Carbocationic Polymerization (1982), page 229, John Wiley & Sons, New York, list several chain transfer agents and their transfer coefficients. Particularly suitable CTAs for the present reaction are selected from the group consisting of 2,4,4-Trimethyl-1-pentene ("α-DIB"), 2.4.4.-Trimethyl-2-pentene ("β-DIB"), 2-ethyl-1-hexene, 2-methyl-1-pentene and mixtures thereof. Of these, α-DIB, β-DIB, or mixtures thereof are preferred. The structures of α-DIB and β-DIB are shown below: 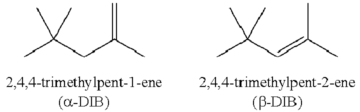 Other CTAs may include 2-octene; 2,5-dimethyl-2,4-hexadiene; cyclohexadiene; isoprene; piperylene and vinylcyclohexane. In general, the chain transfer agent in an olefinic molecule with a molecular weight higher than isobutene and lower than the low molecular weight polymer product produced in accordance with the invention. The CTA is readily detectable by GPC in the product composition by GPC.

In one useful embodiment, the polyisobutylene useful in the present invention is prepared without the use of a chain transfer agent as described above.

To form the polyisobutylene-substituted phenol compound, an aromatic hydroxyl compound, such as a phenol, is reacted (alkylated) with a polyisobutylene as described above. In some embodiments, the aromatic hydroxy compound used for the alkylation may be selected from phenolic compounds with 1, 2 or 3 OH groups and with or without at least one further substituent. In some embodiments, the substituted phenols are mono-ortho-substituted phenols. Suitable substituents are, for example, C₁-C₂₀-alkyl, C₁-C₂₀-alkoxy or a further polyalkylene radical, in particular polyalkylene radicals based on highly reactive polyisobutylenes. Particularly suitable substituents are C₁-C₇-alkyl, such as methyl, ethyl, n-propyl, isopropyl, n-butyl, sec-butyl, tert-butyl and hexyl. Particularly suitable alkyl-substituted phenols are 2-methylphenol and 2-ethylphenol. Unsubstituted phenol is particularly preferred for the alkylation with polyisobutylenes.

In the alkylation, the phenol is usually used in excess. For example, about a 1.1- to 6-fold, preferably 1.6- to 5-fold, excess, such as a 2-fold or a 4-fold excess, of the phenol is suitable.

In one embodiment of the present process, the phenol is used in excess in the preparation of the polyisobutenylphenol and, after the end of the reaction, the reaction mixture is freed from excess phenol by extraction with solvents, preferably polar solvents, such as water or C₁-C₆-alkanols or mixtures thereof, by stripping, i.e. by passing steam through or, if required, heating of gases, e.g. nitrogen, or by distillation.

The process to prepare the intermediate may be carried out a reaction temperature of 1°C to 52°C, or 5°C to 20°C, or 10°C to 15°C.

Suitable alkylation catalysts are known to a person skilled in the art. For example, protic acids, such as sulfuric acid, phosphoric acid and organic sulfonic acids, e.g. trifluoromethanesulfonic acid, Lewis acids, such as aluminum trihalides, e.g. aluminum trichloride or aluminum tribromide, boron trihalides, e.g. boron trifluoride and boron trichloride, tin halides, e.g. tin tetrachloride, titanium halides, e.g. titanium tetrabromide and titanium tetrachloride, and iron halides, e.g. iron trichloride and iron tribromide, are suitable. Adducts of boron trihalides, in particular boron trifluoride, with electron donors such as alcohols, in particular C₁-C₆-alkanols or phenols, or ethers are preferred. Boron trifluoride etherate is particularly preferred.

The alkylation may be carried out in a liquid medium. For this purpose, the phenol is preferably dissolved in one of the reactants and/or in a solvent, if necessary with heating. In a preferred embodiment, the alkylation is preferably carried out by first melting the phenol or the substituted phenol by supplying heat and then adding a suitable solvent and/or the alkylation catalyst, in particular the boron trihalide adduct. The liquid mixture is then brought to a suitable reaction temperature. In a further preferred embodiment the phenol is first melted and the polyisobutylene and, if required, suitable solvent are added. The liquid mixture thus obtained can be brought to a suitable reaction temperature and the alkylation catalyst can then be added.

Suitable solvents for carrying out this reaction are, for example, hydrocarbons, including but not limited to pentane, hexane and heptane or hydrocarbon mixtures, such as petroleum naphthas having boiling ranges from 35 to 100° C., dialkyl ethers, in particular diethyl ether, and halogenated hydrocarbons, such as dichloromethane or trichloromethane, and mixtures of these solvents.

The reaction may be initiated by adding the catalyst or one of the two reactants, phenol or polyisobutylene. The addition of the component initiating the reaction may be done over a period of from 5 to 300, or even from 10 to 200, or even from 15 to 180, minutes. In some embodiments, it is useful to not have the temperature of the reaction mixture exceeding the above-mentioned temperature ranges. After the end of the addition, the reaction mixture is allowed to continue reacting for preferably from 30 minutes to 24 hours, in particular from 60 minutes to 16 hours, at below 30° C. The reaction conditions are preferably chosen so that at least 85%, in particular at least 90%, particularly preferably at least 95%, of the polyisobutenylphenol form. The polyisobutenyl-substituted phenols thus obtained preferably comprise (where the aromatic hydroxy compound used as the starting material allows) more than 85%, in particular more than 90%, and particularly preferably more than 95%, of isomers whose polyisobutenyl radical is para to the hydroxyl group of the phenol.

Optionally the solvent is removed before further reaction to form any derivatives of the polyisobutylene-substituted phenol compound. The reaction mixture may then be neutralized with calcium hydroxide, followed by addition of diatomaceous earth and ammonia hydroxide to remove residual catalyst. The reaction mixture is filtered and the filtrate heated under vacuum to remove volatiles by distillation

When the aromatic hydroxy compound used for the alkylation as described above allows multiple alkylations, the reaction may be carried out in such a way that the polyisobutenylphenols obtained include little if any product more than monoalkylated by the polyisobutylene.

For example, a polyisobutylene-substituted phenol in accordance with the present invention may have the formula:

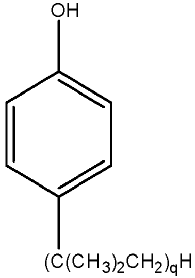

where q is, for example, from 4 to 10, or up to 7, or at least 6, on average, may be formed by reacting phenol with a polyolefin (e.g., polyisobutylene), optionally in the presence of a base catalyst.

### Reaction Scheme 1 illustrates the reaction:

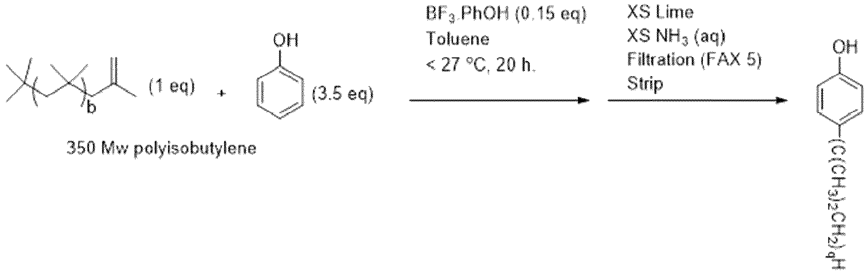

where b is, for example, from 2-8 and q is, for example 4-8.

### Derivatives of the Polyisobutylene-Substituted Hydroxyaromatic Compound

The polyisobutylene-substituted hydroxyaromatic compound in accordance with the present invention may be made into one or more derivatives which have particular application as additives in lubricating composition. Derivatives which may be made using the polyisobutylene-substituted hydroxyaromatic compound, for example, a polyisobutylene-substituted phenol include, polyolefin-substituted bridged hydroxyaromatic compounds, such as phenates and saliginins, Mannich base detergents, oil-soluble zinc salts, polyethers and polyetheramines, methylene coupled hydrocarbyl phenols, and hydrocarbyl substituted amino phenols. These derivatives are described below.

### Polvolefin-Substituted Bridged Hydroxyaromatic Compounds

In one embodiment, the polyisobutylene-substituted hydroxyaromatic phenol of the present invention is used to make a bridged phenol compound in which at least one hydroxyl group is directly bonded to an aromatic ring that is substituted with a polyolefin group.

The aromatic polyol on which the exemplary compound is based may be a substituted or unsubstituted compound that has at least one hydroxyl group directly bonded to an aromatic group (within the definition of Hückel Rule 4π+2 electrons) such as phenol. An exemplary polyolefin-substituted aromatic polyol compound may be represented by the general structure shown below: 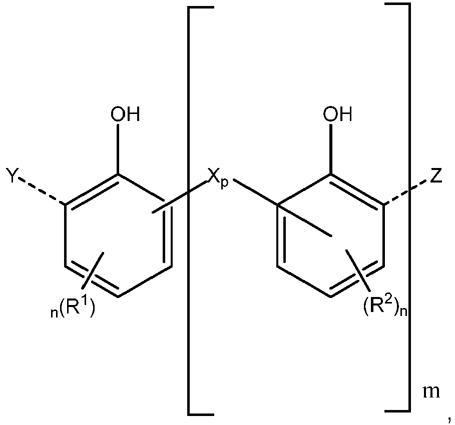 and salts thereof,
where each R¹ and each R² represents a polyolefin group,
X represents a bridging group, such as a sulfur or an alkylene bridging group containing 1 to 8 carbon atoms (e.g., a bridge derived from one or more aldehyde and/or propanal monomer units);
Y and Z each represent a terminal group, such as -H, -OH, a C₁-C₆ alkyl group, or a group derived from the bridging monomer (e.g., -SH, an aldehyde-derived group, such as -C(H)=O, or the like);
each n is at least 1, such as up to 3, on average, or up to 2, or 1;
m is at least 1, such as up to 5, or up to 4, or up to 3, on average, such as 1 or 2; and
p is at least 1, such as up to 5, on average, or 1 or 2. For example, when X is an alkylene bridge, p is 1, 2, or 3, for example, 1 or 2, further for example 1. Further for example, when X is sulfur, p is 1 or 2 or 3.

An exemplary salt may be represented by the general structure shown below: 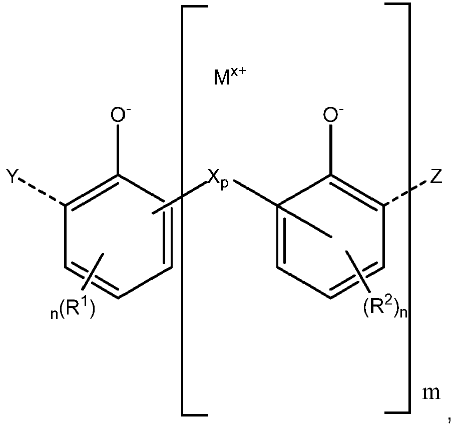 where M represents a cation which is linked to at least one of the O⁻ groups; and x is at least 1, such as 2 in the case of a calcium ion.

The exemplary compound of the above formula may also be associated with a counter ion in the compound. For example, in an overbased compound, the metal ion to compound ratio may be raised above the stoichiometric amount, e.g., by bubbling CO₂ through the mixture to provide a carbonate counterion. As will be appreciated, these aspects can also be used in combinations thereof.

Examples of branched polyolefin groups suitable for use as R¹ and R² include polyolefin groups which are derived from a branched alkene having at least 4 carbon atoms, or up to 12 carbon atoms, or up to 8 carbon atoms, or up to 6 carbon atoms, such as a C₄-C₆ branched alkene. Suitable branched alkenes include isobutylene (2-methylpropene), 2-methylbutene, 2-ethyl-1-butene, 2-methyl-1-pentene, 3-methyl-1-pentene, 4-methyl-1-pentene, 2-methyl, 3-methyl-1-pentene, 2-ethyl-1-pentene, 3-ethyl-1-pentene, 2-methyl-1-hexene, 3-methyl-1-hexene, 2-ethyl-1-hexene, 3-ethyl-1-hexene, 4-ethyl-1-hexene, 2-methyl-1-heptene, 3-methyl-1-heptene, 2-methyl-1-octene, 2-methyl-1-nonene, 2-methyl-1-decene, 2-methyl-1-undecene, and mixtures thereof. Each polyolefin group is derived from at least two or at least three, or at least four, or up to twenty, or up to eighteen, or up to twelve branched alkene monomer units to form a chain with at least two or at least three, or at least four branches from the main chain. In one embodiment, the polyolefin includes a chain derived from at least four, or at least five, or up to eighteen, or up to eight, or up to seven, or up to six branched alkene units. The branched alkene may be branched at the alpha or beta position, or further along the longest chain. In one embodiment, at least one or both of R¹ and R² are derived from a branched alkene which is solely or at least partially isobutylene and at least one or both of R¹ and R² is/are each polyisobutylene (PIB).

R¹ and R² may be independently a polyolefin of 8 to 50 carbon atoms, or at least 10 carbon atoms, or at least 12 carbon atoms, or at least 14 carbon atoms, or at least 16 carbon atoms, or at least 18 carbon atoms, or at least 20 carbon atoms, or at least 24 carbon atoms, or up to 40 carbon atoms, or up to 35 carbon atoms, or up to 30 carbon atoms.

The polyolefin group may have a number-average molecular weight Mₙ of at least 150, or at least 200, or at least 300, or up to 800, or up to 600, or up to 500, or up to 400, or up to 360. In some embodiments R¹ and R² may have different chain lengths. In the exemplary embodiment, R¹ and R² are of the same chain length (or have the same number of branches off the main chain or differ by no more than ±1 branch). A polyolefin group with Mₙ of less than 500, e.g., up to about 400 is particularly suitable as it allows the compound to provide good detergent properties for deposit control and cleanliness without resulting in viscosity creep or undesirable thickening of the oil.

In one embodiment, the compound may be a mixture of polyolefin-substituted, bridged hydroxyaromatic compounds with high and low molecular weight polyolefin groups, respectively. The low molecular weight polyolefin may be up to a C₃₀ or up to a C₂₅ polyolefin. The high molecular weight polyolefin may be up to a C₄₀ or up to a C₃₅ polyolefin. A ratio of the compound with low molecular weight polyolefin to the compound with high molecular weight polyolefin may be at least 50:50 by weight, or at least 80:20, or at least 90:10.

In one embodiment, the compound is free of C₈ and higher unbranched alkyl groups.

In one embodiment, R¹ and R² are each composed solely of carbon and hydrogen.

In one embodiment, the compound consists solely of elements selected from the group consisting of C, H, O, S and the counterion(s).

The compound may have a weight average molecular weight Mw of at least 550, or at least 700, or at least 750, or at least 800 in its unsalted form. The weight average molecular weight of the compound may be up to 5600, or up to 2800, or up to 1200, or up to 1000, or up to 950, in its unsalted form.

In one embodiment X is an alkylene, e.g., a methylene bridge, or a sulfur bridge. In the case of an alkylene bridge, the bridge may be up to 4 carbons in length, or up to 3 carbons in length.

As will be appreciated, these aspects can also be used in combinations thereof.

The salt of the polyisobutylene-substituted bridged phenol compound shown above may be formed by reacting a cation or source of the cation with the compound. The polyisobutylene-substituted bridged phenol compound thus serves as the anion (or "substrate") in the salt. The cation or source thereof reacts with one or more of the residual OH groups to form a neutral or overbased salt of the above-described coupled polyolefin-substituted aromatic polyol.

In one embodiment, the cation is a metallic cation. The metallic cation may be derived from an alkaline earth metal, such as calcium, barium or magnesium (typically calcium), or an alkali metal, such as sodium or potassium (typically sodium). The metal cation may have an atomic weight of at least 6 or at least 12.

Exemplary metal cations include alkali metal cations, alkaline earth metal cations, transition metal cations, and combinations thereof. Examples of metal cations include Li⁺, Na⁺, K⁺, Rb⁺, Cs⁺, Be²⁺, Mg²⁺, Ca²⁺, Sr²⁺, Ba²⁺, Sc³⁺, Sc²⁺, Sc⁺, Y³⁺, Y²⁺, Y⁺, Ti⁴⁺, Ti³⁺, Ti²⁺, Zr⁴⁺, Zr³⁺, Zr²⁺, Hf⁴⁺, Hf³⁺, V⁴⁺, V³⁺, V²⁺, Nb⁴⁺, Nb³⁺, Nb²⁺, Ta⁴⁺, Ta³⁺, Ta²⁺, Cr⁴⁺, Cr³⁺, Cr²⁺, Cr⁺, Mo⁴⁺, Mo³⁺, Mo²⁺, Mo⁺, W⁴⁺, W³⁺, W²⁺, W⁺, Mn⁴⁺, Mn³⁺, Mn²⁺, Mn⁺, Re⁴⁺, Re³⁺, Re²⁺, Re⁺, Fe⁶⁺, Fe⁴⁺, Fe³⁺, Fe²⁺, Fe⁺, Ru⁴⁺, Ru³⁺, Ru²⁺, Os⁴⁺, Os³⁺, Os²⁺, Os⁺, Co⁵⁺, Co⁴⁺, Co³⁺, Co²⁺, Co⁺, Rh⁴⁺, Ru³⁺, Rh²⁺, Rh⁺, Ir⁴⁺, Ir³⁺, Ir²⁺, Ir⁺, Ni³⁺, Ni²⁺, Ni⁺, Pd⁴⁺, Pd²⁺, Pd⁺, Pt⁴⁺, Pt³⁺, Pt²⁺, Pt⁺, Cu⁴⁺, Cu³⁺, Cu²⁺, Cu⁺, Ag³⁺, Ag²⁺, Ag⁺, Au⁴⁺, Au³⁺, Au²⁺, Au⁺, Zn²⁺, Zn⁺, Cd²⁺, Cd⁺, Hg⁴⁺, Hg²⁺, Hg⁺, Al³⁺, Al²⁺, Al⁺, Ga³⁺, Ga⁺, In³⁺, In²⁺, Tl³⁺, Tl⁺ , Si⁴⁺, Si³⁺, Si²⁺, Si⁺, Ge⁴⁺, Ge³⁺, Ge²⁺, Ge⁺, Sn⁴⁺, Sn²⁺, Pb⁴⁺, Pb²⁺, As³⁺, As²⁺, As⁺, Sb³⁺, Bi³⁺, Te⁴⁺, Te²⁺, La³⁺, La²⁺, Ce⁴⁺, Ce³⁺, Ce²⁺, Pr⁴⁺, Pr³⁺, Pr²⁺, Nd³⁺, Nd²⁺, Sm³⁺, Sm²⁺, Eu³⁺, Eu²⁺, Gd³⁺, Gd²⁺, Gd⁺, Tb⁴⁺, Tb³⁺, Tb²⁺, Tb⁺, Db³⁺, Db⁺⁺, Ho³⁺, Er³⁺, Tm⁴⁺, Tm³⁺, Tm²⁺, Yb³⁺, Yb²⁺, and Lu³⁺. Particularly useful are those which form stable salts, i.e., which do not decompose by more than a minor amount over the expected lifetime and operating conditions of the lubricating composition.

In one embodiment, the metallic cation is derived from a metal base such as a metal base of a hydroxide, an oxide, carbonate, or bicarbonate. The metal base may be a hydroxide or an oxide. For example the metallic cation may be derived from calcium hydroxide, calcium oxide, sodium hydroxide, sodium oxide, magnesium hydroxide, magnesium oxide, or mixture thereof.

In one embodiment, the cation is an ash-free cation. An ash-free (ashless) organic cation is an organic ion that does not contain ash-forming metals. In one embodiment, the compound in the salt form has a sulfated ash of up to 0.5 wt. %, or up to 0.4 wt. %, according to ASTM D874.

In one embodiment, the cation is a pnictogen cation. As used herein the term "pnictogens" includes the elements in column 15 of the periodic table. The non-metallic pnictogens include nitrogen and phosphorus (typically nitrogen). The pnictogen cation may be derived from a source of the cation containing a primary amine, a secondary amine, a tertiary amine, or mixture thereof. In one embodiment, the amine salt may be derived from a secondary or tertiary amine.

When the cation is pnictogen cation derived from an amine or ammonium compound, the pnictogen cation (or the amine from which it is derived) may have molecular weight of at least 260 g/mol, or at least 300 g/mol or at least 350 g/mol, or at least 500 g/mol.

The pnictogen cation may be derived from a mono-, di-, or trisubstituted amine. Specific examples include primary alkylamines, such as methylamine, ethylamine, n-propylamine, n-butylamine, n-hexylamine, n-octylamine, 2-ethylhexylamine, benzylamine, 2-phenylethylamine, cocoamine, oleylamine, and tridecylamine (CAS# 86089-17-0); secondary and tertiary alkylamines such as isopropylamine, sec-butylamine, t-butylamine, cyclopentylamine, cyclohexylamine, and 1-phenylethylamine; dialkylamines, such as dimethylamine, diethylamine, dipropylamine, diisopropylamine, dibutylamine, dicyclohexylamine, di-(2-ethylhexyl)amine, dihexylamine, ethylbutylamine, N-ethylcyclohexylamine, and N-methylcyclohexylamine; cycloalkylamines, such as piperidine, N-ethylpiperidine, N,N'-dimethylpiperazine, morpholine, N-methylmorpholine, N-ethylmorpholine, N-methylpiperidine, pyrrolidine, N-methylpyrrolidine, and N-ethylpyrrolidine; and trialkylamines, such as trimethylamine, triethylamine, tripropylamine, triisopropylamine, tri-n-butylamine, trihexylamine, N,N-dimethylbenzylamine, dimethylethylamine, dimethylisopropylamine, dimethylbutylamine, and N,N-dimethylcyclohexylamine.

When the pnictogen cation includes at least one hydrocarbyl group (a quaternary ammonium ion), the pnictogen cation may be an ashless organic cation. Example ammonium cations of this type include N-substituted long chain alkenyl succinimides and aliphatic polyamines. N-substituted long chain alkenyl succinimides useful herein may be derived from an aliphatic polyamine, or mixture thereof. The aliphatic polyamine may be aliphatic polyamine such as an ethylenepolyamine, a propylenepolyamine, a butylenepolyamine, or mixture thereof. Examples of N-substituted long chain alkenyl succinimides include polyisobutylene succinimide with number average molecular weight of the polyisobutylene substituent of at least 350, or at least 500, or at least 550, or at least 750, and can be up to 5000, or up to 3000, or up to 2500. Such succinimides can be formed, for example, from high vinylidene polyisobutylene and maleic anhydride.

Example N-substituted long chain alkenyl succinimides useful herein as pnictogen cations include those derived from succinimide dispersants, which are more fully described in U.S. Pat. Nos. 3,172,892, 3,219,666, 3,316,177, 3,340,281, 3,351,552, 3,381,022, 3,433,744, 3,444,170, 3,467,668, 3,501,405, 3,542,680, 3,576,743, 3,632,511, 4,234,435, RE 26,433, 6,165,235, 7,238,650, and EP Patent Application 0 355 895 A.

Example aliphatic polyamines useful as the pnictogen cation include ethylenepolyamines, propylenepolyamines, butylenepolyamines, and mixtures thereof. Example ethylenepolyamines include ethylenediamine, diethylenetriamine, triethylenetetramine, tetraethylenepentamine, pentaethylenehexamine, polyamine still bottoms, and mixtures thereof.

In one embodiment, the exemplary polyolefin-substituted bridged hydroxyaromatic compound salt may be overbased, i.e., contain an excess of the metal cation in relation to the number of hydroxyl groups present in the compound. There are two common measures of basicity that are commonly used in the field of lubricant additives: Total Base Number (TBN), as measured by ASTM D2896, is a titration that measures both strong and weak bases, while ASTM D4739-11 "Standard Test Method for Base Number Determination by Potentiometric Hydrochloric Acid Titration," (BN) is a titration that measures strong bases but does not readily titrate weak bases, such as certain amines. TBN and BN are expressed as an equivalent in milligrams of potash per gram of oil (mg of KOH/g).

Total base number (TBN), as used herein, is the quantity of acid, expressed in terms of the equivalent number of milligrams of potassium hydroxide (meq KOH), that is required to neutralize all basic constituents present in 1 gram of a sample of the lubricating oil. The TBN values reported herein are determined according to ASTM Standard D2896-15, "Standard Test Method for Base Number of Petroleum Products by Potentiometric Perchloric Acid Titration" (2015), ASTM International, West Conshohocken, PA, 2003 (hereinafter, "D2896"). In various aspects, the neutral salt compound has a TBN of at least 50 mg of KOH/g, or at least 60 mg of KOH/g on an oil-free basis. The TBN of the neutral salt may be up to 300 mg KOH/g, or up to 250 mg KOH/g, or up to 165 mg KOH/g, on an oil-free basis. In various aspects, the lubricating composition containing the compound has a TBN of at least 3 mg KOH/g, or at least 4 mg of KOH/g, or at least 6 mg of KOH/g.

The cation may serve as a basic component of the lubricating composition which, in combination with any other basic components of the lubricating composition, may provide the lubricating composition with a TBN of at least 5, or at least 8, or at least 10, or at least 15, or at least 25. The cation itself may have a TBN of at least 10 or at least or at least 15, or at least 25, or at least 50.

The bridged (e.g., sulfur-coupled or formaldehyde-coupled) polyisobutylene-substituted phenol compound as shown in the above formulas and as described above may be formed through well-known techniques.

In one embodiment, the salt of a polyolefin-substituted hydroxyaromatic compound may be obtained/obtainable by (i) reacting a hydroxy aromatic compound (e.g., phenol) with an branched alkene, such as isobutylene, optionally in the presence of a catalyst, to form a hydroxy-substituted intermediate compound, (ii) coupling the intermediate compound, e.g., with sulfur or formaldehyde, and (to form the salt) iii) reacting the bridged compound formed in step (ii) with a metal base or a pnictogen base.

In one embodiment, sulfurization of the polyisobutylene-substituted phenol intermediate compound may be performed by contacting the intermediate compound with a sulfur source which introduces Sₓ bridging groups between the polyolefin-substituted phenol compound in the presence of a base. The reaction scheme below illustrates the reaction: 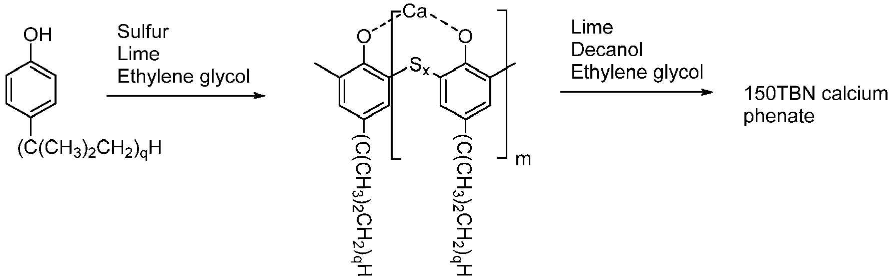 where x is from 1-5, on average, such as up to 3, or up to 2, on average and q is 4-10.

Any suitable sulfur source can be used such as, for example, elemental sulfur or a halide thereof such as sulfur monochloride, sulfur dichloride, hydrogen sulfide, sulfur dioxide, or a sodium sulfide hydrate. The sulfur can be employed either as molten sulfur or as a solid (e.g., powder or particulate) or as a solid suspension in a compatible hydrocarbon liquid, such as ethylene glycol.

Sulfur may be employed at from 0.5 to 4 moles per mole of the intermediate compound in the reaction system. In one embodiment, sulfur is employed at from 0.8 to 2 moles per mole of the intermediate compound.

The temperature range in which the sulfurization reaction is carried out is generally 80-250°C, e.g., 100-220°C. The reaction can be conducted under atmospheric pressure (or slightly lower) or at elevated pressures. During sulfurization a significant amount of by-product hydrogen sulfide gas is evolved. In one embodiment the reaction is carried out under vacuum to facilitate the H₂S elimination.

Other sulfurization techniques which may be adapted to use herein are described, for example, in U.S. Pat. Nos. 2,680,096, to Walker et al., issued Jun. 1, 1954; 3,372,116, to Meinhardt, issued Mar. 6, 1968; 3,036,971, to Otto, issued May 29, 1962, 7,435,709, to Stonebraker, et al., issued Oct. 14, 2008, 8772209 to Mahieux, et al., issued July 8, 2014, 9,062,271 to Jukes, et al., issued June 23, 2015, and U.S. Pub. No. U.S. Pub. No. 20150045269, published February 12, 2015, to Walker, et al. The 20150045269 publication, for example, describes preparation of a sulfurized alkaline earth metal (e.g., calcium) dodecylphenate by reacting dodecylphenol with calcium hydroxide or calcium oxide and an alkylene glycol. The reaction product is reacted with sulfur.

The sulfurization reaction is carried out in the presence of a base, which in one embodiment is the cation source, as described below.

In other embodiments the intermediate compound is contacted with formaldehyde or other aldehyde, which introduces alkylene bridging groups between polyolefin-substituted phenols in the presence of a base.

In general, sulfur coupling produces a more acidic compound which makes over-basing easier.

Formation of the salt may be performed by reaction of the sulfurized polyolefin-substituted compound or other sulfurized intermediate compound or alkylene bridged polyolefin-substituted compound with a basic metal compound which serves as a cation source, such as lime (calcium hydroxide/oxide) or magnesium oxide, or with a pnictogen base, in approximately equimolar amounts, with respect to the OH groups in the intermediate compound, optionally in the presence of a solvent.

Suitable basic metal compounds include hydroxides, oxides and alkoxides of the metal such as (1) an alkali metal salt derived from a metal base selected from an alkali hydroxide, alkali oxide or an alkali alkoxide, or (2) an alkaline earth metal salt derived from a metal base selected from an alkaline earth hydroxide, alkaline earth oxide or alkaline earth alkoxide. Representative examples of metal basic compounds with hydroxide functionality include lithium hydroxide, potassium hydroxide, sodium hydroxide, magnesium hydroxide, calcium hydroxide, barium hydroxide, aluminum hydroxide and the like. Representative examples of metal basic compounds with oxide functionality include lithium oxide, magnesium oxide, calcium oxide, barium oxide and the like. In one embodiment, the alkaline earth metal base is slaked lime (calcium hydroxide).

The pnictogen cation may be derived/derivable from a compound with a primary amine, a secondary amine, a tertiary amine, or mixtures thereof. Typically the amine salt may be derived from a secondary or a tertiary amine.

The amine that can be used to prepare a pnictogen may be any amine capable of salting with a protic acid. The amine may be an alkyl amine, typically a di- or tri- alkyl amine. The alkyl amine may have alkyl groups having 1 to 30, or 2 to 20, or 3 to 10 carbon atoms. Examples of a dialkyl amines include diethylamine, dipropylamine, dibutylamine, dipentylamine, dihexylamine, di-(2-ethylhexyl)amine, di-decylamine, di-dodecylamine, di-stearylamine, dioleylamine, di-eicosylamine, or mixtures thereof. Examples of a trialkyl amine include triethylamine, tripropylamine, tributylamine, tripentylamine, trihexylamine, tri-(2-ethylhexyl)amine, tri-decylamine, tri-dodecylamine, tri-stearylamine, trioleylamine, tri-eicosylamine, and mixtures thereof.

The amine may also be a tertiary-aliphatic primary amine. The aliphatic group in this case may be an alkyl group containing 2 to 30, or 6 to 26, or 8 to 24 carbon atoms. Tertiary alkyl amines include monoamines such as tert-butylamine, tert-hexylamine, 1-methyl-1-amino-cyclohexane, tert-octylamine, tert-decylamine, tert-dodecylamine, tert-tetradecylamine, tert-hexadecylamine, tert-octadecylamine, tert-tetracosanylamine, and tert-octacosanylamine.

In one embodiment, the pnictogen base includes a phosphorus acid amine salt which includes an amine with C₁₁ to C₂₂ tertiary alkyl primary groups, or mixtures thereof.

In one embodiment the amine salt may be in the form of a quaternary ammonium salt. Examples of quaternary ammonium salts containing a hydroxyalkyl group, and methods for their synthesis, are disclosed in U.S. Pat No. 3,962,104. In certain embodiments, the quaternary ammonium compound is derived from a monoamine by means of alkylation, i.e., from a tertiary amine having only a single amino group, that is, having no additional amine nitrogen atoms in any of the three hydrocarbyl groups or substituted hydrocarbyl groups attached to the tertiary amine nitrogen. In certain embodiments there are no additional amine nitrogen atoms in any of the hydrocarbyl groups or substituted hydrocarbyl groups attached to the central nitrogen in the quaternary ammonium ion. The tetraalkylammonium hydroxide may contain alkyl groups having 1 to 30, or 2 to 20, or 3 to 10 carbon atoms. The tetraalkylammonium hydroxide may include tetrapropylammonium hydroxide, tetrabutylammonium hydroxide, tetra-pentylammonium hydroxide, tetrahexylammonium hydroxide, tetra-2-ethylhexyl-ammonium hydroxide, tetradecylammonium hydroxide, or a mixture thereof.

The amine may be quaternized with a quaternizing agent, or mixture thereof.

The pnictogen base may include an aminoalkyl substituted heterocyclic compound, such as 1-(3-aminopropyl)imidazole, 4-(3-aminopropyl)morpholine, 1-(2-aminoethyl)piperidine, 3,3-diamino-N-methyldipropylamine, and 3,3-aminobis(N,N-dimethylpropylamine).

Other examples of quaternary ammonium salts and methods for preparing the same are described in U.S. Pat. Nos. 3,778,371, 4,171,959, 4,253,980, 4,326,973, 4,338,206, and 5,254,138.

When the amine salt is derived from an aromatic amine, the aromatic amine may form an ion such as a pyridinium ion, or an imidazolium ion. Certain quaternary phosphonium salts may be prepared by the reaction of phosphine with aldehydes and a halide e.g., tetrakis(hydroxymethyl)phosphonium halide (typically chloride).

A quaternary pnictogen halide compound may be a commercially available material, or it may be prepared by reaction of a tertiary amine with a hydrocarbyl halide, by known techniques. This reaction may be performed in a separate vessel or in the same vessel in which it is subsequently (or simultaneously) reacted with the oil-soluble acidic compound, which may be converted previously (or simultaneously) into its metal neutralized form.

Neutralization of the sulfurized intermediate compound may be carried out in a continuous or batch process by any method known to a person skilled in the art. In general, neutralization can be carried out by contacting the sulfurized or intermediate compound with a metal or pnictogen base under reactive conditions, e.g., in an inert-compatible liquid hydrocarbon diluent. If desired, the reaction can be conducted under an inert gas, such as nitrogen. The metal or pnictogen base may be added either in a single addition or in a plurality of additions at intermediate points during the reaction.

Neutralization may be conducted in a suitable solvent or diluent oil, such as toluene, xylene and commonly with a promoter such as an alcohol, e.g., a C, to C₁₆ alcohol, such as methanol, decyl alcohol, or 2-ethylhexanol; a diol, e.g., C₂ to C₄ alkylene glycols, such as ethylene glycol; and/or carboxylic acids. Suitable diluent oils include naphthenic oils and mixed oils, e.g., paraffinic. The quantity of solvent or diluent oil used may be such that the amount of solvent or oil in the final product constitutes from 15% to 65% by weight of the final product, such as from 25% to 50%.

The neutralization reaction may be conducted at temperatures above room temperature (20°C). In general, neutralization can be carried out at a temperature of between 150-200°C. The neutralization reaction itself may take place for over 5 to 60 minutes up to 9 hours, for example, 7 hours.

In another embodiment, the salt of the polyolefin-substituted bridged hydroxyaromatic compound can be prepared in a one-pot method. In this method, the intermediate compound is combined with diluent oil and ethylene glycol and heated while stirring. A metal or pnictogen base, such as hydrated lime, is added to the heated reaction mixture, e.g., in several portions. Sulfur is added to the reaction mixture, and optionally additional metal or pnictogen base is added and the mixture stirred. The reaction mixture may be vacuum stripped to remove excess solvent.

In one embodiment, the exemplary polyolefin-substituted bridged hydroxyaromatic compound may be overbased. Overbasing can be carried out either during or after one of the sulfurization and/or neutralization steps. Alternatively, sulfurization, neutralization and overbasing can be carried out simultaneously. In general, the overbasing is carried out by reaction of the salt of the sulfur-coupled polyolefin-substituted aromatic polyol with an acidic overbasing compound, such as carbon dioxide or boric acid. In one embodiment, an overbasing process is by way of carbonation, i.e., a reaction with carbon dioxide. Such carbonation can be conveniently effected by addition of solvents such as aromatic solvents, alcohols or a polyols, typically an alkylene diol, e.g., ethylene glycol. Conveniently, the reaction is conducted by bubbling of gaseous carbon dioxide through the reaction mixture, optionally in the presence of sulfonic acid. Excess solvents and any water formed during the overbasing reaction can be conveniently removed by distillation either during or after the reaction.

In one embodiment, the overbasing reaction is carried out in a reactor by reacting the salt of the polyolefin-substituted bridged hydroxyaromatic compound with a source of an alkaline earth metal such as lime (i.e., an alkaline earth metal hydroxide) in the presence of carbon dioxide, and optionally in the presence of an aromatic solvent (e.g., xylene), and/or a hydrocarbyl alcohol, such as methanol. The reaction may be conducted by bubbling gaseous carbon dioxide through the reaction mixture. The carbon dioxide is introduced over a period of 1 hour to 3 hours, at a temperature ranging from 150-200°C. The degree of overbasing may be controlled by the quantity of the source of an alkaline earth metal, carbon dioxide and the reactants added to the reaction mixture and the reaction conditions used during the carbonation process.

In another embodiment, the overbasing reaction can be carried out at from 140-180°C in the presence of a polyol, typically an alkylene diol, e.g., ethylene glycol, and/or alkanols, e.g., C₆ to C₁₆ alkanol(s), such as decyl alcohols or 2-ethyl hexanol. Excess solvent and any water formed during the overbasing reaction can be conveniently removed by distillation either during or after the reaction.

Methods for forming overbased detergents useful herein are described, for example, in U.S. Pat. Nos. 5,259,966, 6,015,778, 5,534,168, and 6,268,318, and U.S. Pub. No. 2013/0203639.

The resulting overbased salt of the polyolefin-substituted bridged hydroxyaromatic compound may contain some amount of unsulfurized hydroxy-substituted intermediate compound and/or its unsulfurized metal salt.

The composition containing the overbased salt of the polyolefin-substituted bridged hydroxyaromatic compound may be sparged, e.g., by bubbling gas, such as air or nitrogen, at a temperature ranging from 190-250°C through the composition. The sparging results in removing substantially all of the unsulfurized hydroxy-substituted intermediate compound and salts thereof to provide a composition substantially free of the unsulfurized hydroxy-substituted intermediate compound and unsulfurized salts thereof. The term "substantially free" as used herein means less than 1.5 wt. %, or less than 1 wt. %, or less than 0.3 wt. % of these unsulfurized compounds, such as 0.1-0.3 wt. %, or less.

In one embodiment, the salt of the polyolefin-substituted bridged hydroxyaromatic compound does not contain any sulfonate functional groups. In one embodiment, the salt of the polyolefin-substituted bridged hydroxyaromatic compound does not contain any phosphate functional groups. In one embodiment, the salt of the polyolefin-substituted bridged hydroxyaromatic compound does not contain any borate functional groups. In another embodiment, the salt of the polyolefin-substituted bridged hydroxyaromatic compound does contain a borate functional group.

The salts described above can be boronated by processes known to those skilled in the art. Boration can be accomplished either prior to, or after, the overbasing step. The boration can be accomplished by a number of boronating agents, such as boric acid, metaboric acid, orthoboric acid, alkyl borates, boron halides, polymers of boron, esters of boron and similar materials. When present, the boron content of the salt may be 0.1 wt. % to 5 wt. %, or 1 wt. % to 5 wt. %, or 2 wt. % to 4 wt. %.

The salt of the polyolefin-substituted bridged hydroxyaromatic compound, in one embodiment, may be formed from an anion composed of carbon, hydrogen, oxygen, boron and nitrogen; and a metallic cation.

In one embodiment, the salt of the polyolefin-substituted bridged hydroxyaromatic compound may comprise or consist of an anion composed of carbon, hydrogen, oxygen and optionally nitrogen; and a metallic cation, such as a calcium, magnesium or sodium cation.

Examples of suitable ethylenically unsaturated esters of boron include(meth)acrylates, fumarates and maleates which are derived from saturated alcohols, such as 2-ethylhexyl (meth)acrylate, heptyl (meth)acrylate, 2-tert-butylheptyl (meth)acrylate, octyl (meth)acrylate, 3-isopropylheptyl (meth)acrylate, nonyl (meth)acrylate, decyl (meth)acrylate, undecyl (meth)acrylate; and the corresponding fumarates and maleates. The expression "(meth)acrylates" encompasses methacrylates and acrylates and also mixtures of the two.

### Mannich Base Detergents

The polyisobutylene-substituted phenol of the present invention can be used in the preparation of a Mannich detergent, sometimes referred to as a Mannich base detergent. Mannich detergent is a reaction product of a hydrocarbyl-substituted phenol, an aldehyde, and an amine or ammonia. The aldehyde used to form the Mannich detergent can have 1 to 10 carbon atoms, and is generally formaldehyde or a reactive equivalent thereof such as formalin or paraformaldehyde. The amine used to form the Mannich detergent can be a monoamine or a polyamine, including alkanolamines having one or more hydroxyl groups. Useful amines include those described above, such as ethanolamine, diethanolamine, methylamine, dimethylamine, ethylenediamine, dimethylaminopropylamine, diethylenetriamine and 2-(2-aminoethylamino) ethanol. The Mannich detergent can be prepared by reacting a hydrocarbyl-substituted phenol, an aldehyde, and an amine as described in U.S. Pat. No. 5,697,988. In one embodiment of this invention the Mannich reaction product is prepared from an alkylphenol derived from a polyisobutylene, formaldehyde, and an amine that is a primary monoamine, a secondary monoamine, or an alkylenediamine, in particular, ethylenediamine or dimethylamine. The Mannich reaction product can be prepared by well known methods generally involving reacting the hydrocarbyl substituted hydroxy aromatic compound, an aldehyde and an amine at temperatures between 50 to 200° C. in the presence of a solvent or diluent while removing reaction water as described in U.S. Pat. No. 5,876,468.

### Oil Soluble Zinc Salts

The polyisobutylene-substituted phenol of the present invention can be use to prepare oil-soluble zinc salts. The hydrocarbyl group of the alkylated phenol provides the required oil solubility, and its particular length or other characteristics may vary depending on the type of zinc salt involved. Suitable zinc salts that can be prepared using the alkylated phenol of the present invention include zinc phosphates, phosphites, phosphonates, sulfonates, phenates, and salicylates.

In one embodiment, the zinc salt is a zinc hydrocarbyl phosphate. The phosphate can be a mono-or dihydrocarbyl phosphate. The hydrocarbyl groups typically each independently contain 1 to 40 carbon atoms, preferably 1 to 30 carbon atoms, provided, as stated above, that at least one hydrocarbyl group contains at least 6 carbon atoms. In a preferred embodiment, each hydrocarbyl is independently an aliphatic or aryl group provided that at least one is an aryl group provided by the alkylated phenol of the present invention.

The zinc hydrocarbyl phosphates can be prepared by reacting phosphorus acid or anhydride, preferably phosphorus pentoxide, with the alkylated phenol at a temperature of 30° C. to 200° C., preferably 80° C. to 150° C., followed by neutralization with a zinc base. The phosphorus acid is generally reacted with the alkylated phenol in a ratio of about 1:3.5, preferably 1:2. The product of such a reaction typically comprises a mixture of monohydrocarbyl and dihydrocarbyl zinc phosphates, typically being present in a relative ratios of about 1:1, or more generally, 2:1 to 1:2 or 3:1 to 1:3. Mixtures of about 1:1 monohydrocarbyl: dihydrocarbyl materials can be prepared by the simple stoichiometric reaction of alcohol with P ₂O ₅:

3ROH+P ₂O ₅→RO--P(=O)--(OH) ₂+(RO) ₂--P(=O)--OH

### Polyethers and Polyetheramines

The polyisobutylene-substituted phenol of the present invention can be used to prepare exemplary polyether compound having two or more ether groups and optionally at least one amino group, which can be a primary, secondary or tertiary amino group.

The polyether and/or polyetheramine is represented by the formula

R[OCH₂CH(R¹)]ₙA,

where R is a hydrocarbyl aryl group, R¹ is hydrogen or a hydrocarbyl group, A is a nitrogen-containing group, in the case of a polyetheramine, or A is a hydroxyl group (OH), in the case of a polyether, and n is a number which is at least 2. R can be derived from an alkylphenol, or a mixture thereof where the mixture can be a mixture of 2 or more alkylphenols, or at least 1 or more alkylphenols and 1 or more alcohols. The number n of alkylene oxide groups in the polyetheramine/polyether formula can be at least 10, or at least 16, or at least 18 and may be up to 50, or up to 38, or up to 28, or up to 26, or up to 24.

"A" in the polyetheramine formula may be selected from amines, ether amines and mixtures thereof. Suitable amines and ether amines include those of the formulas -OCH₂CH₂CH₂NR²R² and -NR³R³, where each R² is independently hydrogen or a hydrocarbyl group of one or more carbon atoms, and each R³ is independently hydrogen, a hydrocarbyl group of one or more carbon atoms, or - [R⁴(R⁵)]_{P}R⁶, where R⁴ is a C₂-C₁₀ alkylene, R⁵ and R⁶ are independently hydrogen or a hydrocarbyl group of one or more carbon atoms, and p is a number from 1 to 7.

The polyetheramine may be derived from a polyether intermediate of the general form RO[CH₂CH(R¹)O]ₙH where R, R¹ and n are as described above. The polyether intermediate can be formed by condensing an alcohol and/or alkylphenol with one or more alkylene oxides in a base catalyzed reaction as described, for example, in U.S. Pat. No. 5,094,667. A ratio of alcohol and/or alkylphenol to alkylene oxide may be from 1 :2 to 1 :50, and in some embodiments, can be at least 1 :10, or at least 1 :16, or at least 1 :18, or up to 1 :38, or up to 1 :28, or up to 1 :26.

The polyether intermediate can be converted to a polyetheramine where A is -NR³R³, as described above, by a direct amination reaction of the polyether intermediate with an amine, ammonia, or a polyamine as described, for example, in EP 0310875, The polyether intermediate can be converted to a polyetheramine where A is -OCH₂CH₂CH₂NR²R² by reaction with acrylonitrile followed by hydrogenation, as described, for example, in U.S. Pat. No. 5,094,667. In one embodiment, the polyether intermediate can be converted to a polyetheramine where A is -OCH₂CH₂CH₂NH₂ by reacting the polyether intermediate with acrylonitrile to form a cyanoethylated intermediate which can then be hydrogenated to form the polyetheramine.

Suitable polyetheramines include those where A is

-OCH₂CH₂CH₂NH₂.

The polyetheramine and/or polyether may have a number average molecular weight of at least 300 or at least 350, or at least 400, or at least 450, or at least 1000, and in some embodiments, the number average molecular weight may be up to 5000, or up to 3500, or up to 2500, or up to 2000. Example polyetheramines/polyethers include butylene oxide-based polyetheramines/polyethers and propylene oxide-based polyetheramines/polyethers where n is 20-24 and R is an alkylphenol of the present invention.

### Methylene coupled hydrocarbyl phenols

The polyisobutylene-substituted phenol of the present invention may be condensed by reaction in the presence of aldehyde, ketone, or reactive equivalent thereof to form hydrocarbyl- and hydroxy-substituted aromatic condensates. Suitable aldehyde or ketone compounds used to condense (b)(1) and (b)(2) may have 1 to 10, 1 to 4 or 1 to 2 carbon atoms. In an embodiment of the invention the aldehyde is formaldehyde or a reactive equivalent thereof including formalin, paraformaldehyde or mixtures thereof. The amount of aldehyde, ketone, or reactive equivalent thereof present to condense the alkylated phenol typically ranges from 50 mol % to 200 mol %, 75 mol % to 150 mol % or 80 mol % to 120 mol % of the hydrocarbyl- and hydroxy-substituted aromatic condensate.

The hydrocarbyl- and hydroxy-substituted aromatic condensate may be prepared by a process comprising reacting:
(1) 0.1 to 100 mole percent based on the total amount of a hydrocarbyl- and hydroxy-substituted aromatic compound of the present invention wherein the hydrocarbyl substituent is a branched substituent containing 20 to 40 carbon atoms;
(2) 0 to 99.9 mole percent based on the total amount of a hydrocarbyl- and hydroxy-substituted aromatic compound wherein the hydrocarbyl substituent is an alkyl phenol other than that of the present invention
(3) an amount of an aldehyde, ketone, or reactive equivalent thereof effective to condense (1) and (2) to form the hydrocarbyl- and hydroxy-substituted aromatic condensate.

The hydrocarbyl- and hydroxy-substituted aromatic condensate may be prepared at a reaction temperature ranging from 50° C. to 200° C., 60 C to 175° C., or 75° C. to 140° C.; the total reaction time may be from 1 minute to 24 hours, 1 hour to 18 hours or 2 hours to 12 hours. In one embodiment the reaction is carried out under reduced pressure or in an inert atmosphere, such as under nitrogen atmosphere including with a nitrogen sparge. The hydrocarbyl- and hydroxy-substituted aromatic condensate, may also be prepared by standard techniques used to prepare an alkyl phenol condensate as described in EP 0311452 (sec for example, the techniques employed in Examples 1 to 22).

### Hydrocarbyl-Substituted Amino Phenols

The polyisobutylene-substituted phenol of the present invention can also be used to prepare a hydrocarbyl-substituted aminophenol. The hydrocarbyl-substituted aminophenol can have one or more hydrocarbyl substituents but generally has a single hydrocarbyl substituent. The hydrocarbyl-substituted aminophenol can have one or more amino groups, in another instance can have two amino groups, and in a further instance can have a single amino group. The amino group of the aminophenol can be represented by the formula -NH₂. The hydrocarbyl-substituted aminophenol can be prepared by nitrating the alkylated phenol of the present invention with a nitrating agent such as nitric acid, and reducing the nitrated phenol with a reducing agent such as hydrazine at temperatures of 100 to 200° C. or with a metal catalyzed hydrogenation as described in U.S. Pat. No. 4,724,091.

### Lubricating Compositions

The polyisobutylene-substitued phenol and the derivatives thereof may be present in in a lubricating composition at a concentration of at least 0.1 wt. % and may be up to 20 wt. %. For example, the concentration of the compound may be at least 0.25 wt. %, or at least 0.5 wt. %, or at least 1 wt. %, or at least 5 wt. %, or at least 10 wt. %, or at least 15 wt. % of the lubricating composition. The compound may also be present in a concentrate, alone or with other additives and with a lesser amount of oil. In a concentrate, the amount of the compound may be at least 2, or at least 3 times the concentration in the lubricating composition.

In addition to the polyisobutylene-substituted phenol compound or derivatives thereof, the exemplary lubricating composition includes an oil of lubricating viscosity and optionally one or more additional performance additives suited to providing the performance properties of a fully formulated lubricating compositions. The polyisobutylene-substituted phenol and derivatives thereof of the present invention may be useful in a variety of lubricating applications, including but not limited to engine oils, hydraulic oils, industrial gear oils, metalworking fluids, refrigerant lubricants, driveline fluids, and transmission fluids. Examples of these additional performance additives include (overbased) detergents, viscosity modifiers, friction modifiers, antioxidants, dispersants, antiwear/antiscuffing agents, metal deactivators, extreme pressure agents, foam inhibitors, demulsifiers, pour point depressants, corrosion inhibitors, seal swelling agents, and the like, which may be used singly or in combination.

### Oil of lubricating viscosity

The lubricating composition may include the oil of lubricating viscosity as a minor or major component thereof, such as at least 5 wt. %, or at least 10 wt. %, or at least 20 wt. %, or at least 30 wt. %, or at least 40 wt. %, or at least 60 wt. %, or at least 80 wt. % of the lubricating composition.

Suitable oils include natural and synthetic oils, oil derived from hydrocracking, hydrogenation, and hydrofinishing, unrefined, refined, re-refined oils or mixtures thereof. Unrefined, refined and re-refined oils, and natural and synthetic oils are described, for example, in WO2008/147704 and US Pub. No. 2010/197536. Synthetic oils may also be produced by Fischer-Tropsch reactions and typically may be hydroisomerized Fischer-Tropsch hydrocarbons or waxes. Oils may be prepared by a Fischer-Tropsch gas-to-liquid synthetic procedure as well as other gas-to-liquid procedures.

Oils of lubricating viscosity may also be defined as specified in April 2008 version of "Appendix E - API Base Oil Interchangeability Guidelines for Passenger Car Motor Oils and Diesel Engine Oils", section 1.3 Sub-heading 1.3. "Base Stock Categories". The API Guidelines are also summarized in US Pat. No. 7,285,516. The five base oil groups are as follows: Group I (sulfur content >0.03 wt. %, and/or <90 wt. % saturates, viscosity index 80-120); Group II (sulfur content <0.03 wt. %, and ≥90 wt. % saturates, viscosity index 80-120); Group III (sulfur content <0.03 wt. %, and ≥90 wt. % saturates, viscosity index ≥120); Group IV (all polyalphaolefins (PAOs)); and Group V (all others not included in Groups I, II, III, or IV). The exemplary oil of lubricating viscosity includes an API Group I, Group II, Group III, Group IV, Group V oil, or mixtures thereof. In some embodiments, the oil of lubricating viscosity is an API Group I, Group II, Group III, or Group IV oil, or mixtures thereof. In some embodiments, the oil of lubricating viscosity is an API Group I, Group II, or Group III oil, or mixture thereof. In one embodiment the oil of lubricating viscosity may be an API Group II, Group III mineral oil, a Group IV synthetic oil, or mixture thereof. In some embodiments, at least 5 wt. %, or at least 10 wt. %, or at least 20 wt. %, or at least 40 wt. % of the lubricating composition is a polyalphaolefin (Group IV).

The oil of lubricating viscosity may have a kinematic viscosity of up to 30 mm²/s or up to 25 mm²/s (cSt) at 100 °C and can be at least 12 mm²/s at 100 °C, and in other embodiments at least 15 mm²/s. As used herein, kinematic viscosity is determined at 100 °C by ASTM D445-14, "Standard Test Method for Kinematic Viscosity of Transparent and Opaque Liquids (and Calculation of Dynamic Viscosity)," ASTM International, West Conshohocken, PA, 2003, DOI: 10.1520/D0445-14 and may be referred to as KV_100.

In certain embodiments, the lubricating composition may contain synthetic ester base fluids. Synthetic esters may have a kinematic viscosity measured at 100°C of 2.5 mm²/s to 30 mm²/s. In one embodiment, the lubricating composition comprises less than 50 wt. % of a synthetic ester base fluid with a KV_100 of at least 5.5 mm²/s, or at least 6 mm²/s, or at least 8 mm²/s.

Exemplary synthetic oils include poly-alpha olefins, polyesters, polyacrylates, and poly-methacrylates, and co-polymers thereof. Example synthetic esters include esters of a dicarboxylic acid (e.g., selected from phthalic acid, succinic acid, alkyl succinic acids, alkenyl succinic acids, maleic acid, azelaic acid, suberic acid, sebacic acid, fumaric acid, adipic acid, linoleic acid dimer, malonic acid, alkyl malonic acids, and alkenyl malonic acids) with an alcohol (e.g., selected from butyl alcohol, hexyl alcohol, dodecyl alcohol, 2-ethylhexyl alcohol, ethylene glycol, diethylene glycol monoether, and propylene glycol). Specific examples of these esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, the 2-ethylhexyl diester of linoleic acid dimer, and the complex ester formed by reacting one mole of sebacic acid with two moles of tetraethylene glycol and two moles of 2-ethylhexanoic acid.

Esters useful as synthetic oils also include those made from C₅ to C₁₂ monocarboxylic acids and polyols and from polyol ethers such as neopentyl glycol, trimethylolpropane, pentaerythritol, dipentaerythritol, and tripentaerythritol. Esters can also be monoesters, such as are available under the trade name Priolube 1976^{™} (C₁₈-alkyl-COO-C₂₀ alkyl).

Synthetic ester base oils may be present in the lubricating composition of the invention in an amount less than 50 wt. % of the composition, or less than 40 weight %, or less than 35 weight %, or less than 28 weight %, or less than 21 weight %, or less than 17 weight %, or less than 10 weight %, or less than 5 weight % of the composition. In one embodiment, the lubricating composition of the invention is free of, or substantially free of, a synthetic ester base fluid having a KV_100 of at least 5.5 mm²/s.

Example natural oils include animal and vegetable oils, such as long chain fatty acid esters. Examples include linseed oil, sunflower oil, sesame seed oil, beef tallow oil, lard oil, palm oil, castor oil, cottonseed oil, corn oil, peanut oil, soybean oil, olive oil, whale oil, menhaden oil, sardine oil, coconut oil, palm kernel oil, babassu oil, rape oil, and soya oil.

The amount of the oil of lubricating viscosity present is typically the balance remaining after subtracting from 100 weight % the sum of the amount of the exemplary amino-carboxylate compound and the other performance additives.

### Other Additives

### Detergents

The lubricating composition optionally further includes at least one detergent, other than the exemplary compound. Exemplary detergents useful herein include overbased metal-containing detergents. The metal of the metal-containing detergent may be zinc, sodium, calcium, barium, or magnesium. The overbased metal-containing detergent may be chosen from sulfonates, non-sulfur containing phenates, sulfur containing phenates, salixarates, salicylates, and mixtures thereof, or borated equivalents thereof. The overbased detergent may be borated with a borating agent such as boric acid.

The overbased metal-containing detergent may also include "hybrid" detergents formed with mixed surfactant systems including phenate and/or sulfonate components, e.g., phenate/salicylates, sulfonate/phenates, sulfonate/salicylates, sulfonates/phenates/salicylates, as described, for example, in U.S. Pat. Nos. 6,429,178; 6,429,179; 6,153,565; and 6,281,179. Where a hybrid sulfonate/phenate detergent is employed, the hybrid detergent can be considered equivalent to amounts of distinct phenate and sulfonate detergents introducing like amounts of phenate and sulfonate soaps, respectively.

Example overbased metal-containing detergents include zinc, sodium, calcium and magnesium salts of sulfonates, phenates (including sulfur-containing and non-sulfur containing phenates), salixarates and salicylates. Such overbased sulfonates, salixarates, phenates and salicylates may have a total base number of 120 to 700, or 250 to 600, or 300 to 500 (on an oil free basis).

The overbased sulfonate detergent may have a metal ratio of 12 to less than 20, or 12 to 18, or 20 to 30, or 22 to 25.

Typically, an overbased metal-containing detergent may be a zinc, sodium, calcium or magnesium salt of a sulfonate, a phenate, sulfur containing phenate, salixarate or salicylate. Overbased sulfonates, salixarates, phenates and salicylates typically have a total base number of 120 to 700 TBN. Overbased sulfonates typically have a total base number of 120 to 700, or 250 to 600, or 300 to 500 (on an oil free basis).

The overbased sulfonate detergent may have a metal ratio of 12 to less than 20, or 12 to 18, or 20 to 30, or 22 to 25.

Example sulfonate detergents include linear and branched alkylbenzene sulfonate detergents, and mixtures thereof, which may have a metal ratio of at least 8, as described, for example, in U.S. Pub. No. 2005065045. Linear alkyl benzenes may have the benzene ring attached anywhere on the linear chain, usually at the 2, 3, or 4 position, or be mixtures thereof. Linear alkylbenzene sulfonate detergents may be particularly useful for assisting in improving fuel economy.

In one embodiment, the alkylbenzene sulfonate detergent may be a branched alkylbenzene sulfonate, a linear alkylbenzene sulfonate, or mixtures thereof.

In one embodiment, the lubricating composition may be free of linear alkylbenzene sulfonate detergent. The sulfonate detergent may be a metal salt of one or more oil-soluble alkyl toluene sulfonate compounds as disclosed in U.S. Pub. No. 20080119378.

The lubricating composition may include at least 0.01 wt. % or at least 0.1 wt. % of the detergent other than the exemplary compound, and in some embodiments, up to 2 wt. %, or up to 1 wt. % detergent.

### Antioxidants

The lubricating composition optionally further includes at least one antioxidant. Exemplary antioxidants useful herein include phenolic and aminic antioxidants, such as diarylamines, alkylated diarylamines, hindered phenols, and mixtures thereof. The diarylamine or alkylated diarylamine may be a phenyl-α-naphthylamine (PANA), an alkylated diphenylamine, an alkylated phenylnapthylamine, or mixture thereof. Example alkylated diphenylamines include dinonyl diphenylamine, nonyl diphenylamine, octyl diphenylamine, dioctyl diphenylamine, didecyl diphenylamine, decyl diphenylamine, and mixtures thereof. Example alkylated diarylamines include octyl, dioctyl, nonyl, dinonyl, decyl and didecyl phenylnapthylamines. Hindered phenol antioxidants often contain a secondary butyl and/or a tertiary butyl group as a steric hindering group. The phenol group may be further substituted with a hydrocarbyl group (e.g., a linear or branched alkyl) and/or a bridging group linking to a second aromatic group. Examples of suitable hindered phenol antioxidants include 2,6-di-tertbutylphenol, 4-methyl-2,6-di-tert-butylphenol, 4-ethyl-2,6-di-tert-butylphenol, 4-propyl-2,6-di-tert-butylphenol, 4-butyl-2,6-di-tert-butylphenol, and 4-dodecyl-2,6-di-tert-butylphenol. In one embodiment, the hindered phenol antioxidant may be an ester, such as those described in U.S. Pat. No. 6,559,105. One such hindered phenol ester is sold as Irganox^{™} L-135, obtainable from Ciba.

When present, the lubricating composition may include at least 0.1 wt. % or at least 0.5 wt. %, or at least 1 wt. % antioxidant, and in some embodiments, up to 3 wt. %, or up to 2.75 wt. %, or up to 2.5 wt. % antioxidant.

### Dispersants

The lubricating composition optionally further includes at least one dispersant, other than the exemplary compound. Exemplary dispersants include succinimide dispersants, Mannich dispersants, succinamide dispersants, and polyolefin succinic acid esters, amides, and ester-amides, and mixtures thereof. The succinimide dispersant, where present, may be as described above for the succinimides described as useful for cation M.

The succinimide dispersant may be derived from an aliphatic polyamine, or mixtures thereof. The aliphatic polyamine may be an ethylenepolyamine, a propylenepolyamine, a butylenepolyamine, or a mixture thereof. In one embodiment the aliphatic polyamine may be an ethylenepolyamine. In one embodiment the aliphatic polyamine may be chosen from ethylenediamine, diethylenetriamine, triethylenetetramine, tetraethylenepentamine, pentaethylenehexamine, polyamine still bottoms, and mixtures thereof.

In one embodiment, the dispersant may be a polyolefin succinic acid ester, amide, or ester-amide. A polyolefin succinic acid ester-amide may be a polyisobutylene succinic acid reacted with an alcohol (such as pentaerythritol) and a polyamine as described above. Example polyolefin succinic acid esters include polyisobutylene succinic acid esters of pentaerythritol and mixture thereof.

The dispersant may be an N-substituted long chain alkenyl succinimide. An example of an N-substituted long chain alkenyl succinimide is polyisobutylene succinimide. Typically the polyisobutylene from which polyisobutylene succinic anhydride is derived has a number average molecular weight of 350 to 5000, or 550 to 3000 or 750 to 2500. Succinimide dispersants and their preparation are disclosed, for example, in US Pat. Nos. 3,172,892, 3,219,666, 3,316,177, 3,340,281, 3,351,552, 3,381,022, 3,433,744, 3,444,170, 3,467,668, 3,501,405, 3,542,680, 3,576,743, 3,632,511, 4,234,435, Re 26,433, and 6,165,235, and 7,238,650 and EP Patent Application 0 355 895 A.

The succinimide dispersant may comprise a polyisobutylene succinimide, wherein the polyisobutylene from which polyisobutylene succinimide is derived has a number average molecular weight of 350 to 5000, or 750 to 2500.

The exemplary dispersants may also be post-treated by conventional methods by a reaction with any of a variety of agents. Among these are boron compounds (such as boric acid), urea, thiourea, dimercaptothiadiazoles, carbon disulfide, aldehydes, ketones, carboxylic acids, such as terephthalic acid, hydrocarbon-substituted succinic anhydrides, maleic anhydride, nitriles, epoxides, and phosphorus compounds. In one embodiment the post-treated dispersant is borated. In one embodiment the post-treated dispersant is reacted with dimercaptothiadiazoles. In one embodiment the post-treated dispersant is reacted with phosphoric or phosphorous acid. In one embodiment the post-treated dispersant is reacted with terephthalic acid and boric acid (as described in U.S. Pub. No. 2009/0054278.

When present, the lubricating composition may include at least 0.01 wt. %, or at least 0.1 wt. %, or at least 0.5 wt. %, or at least 1 wt. % of other dispersant(s), and in some embodiments, up to 20 wt. %, or up to 15 wt. %, or up to 10 wt. %, or up to 6 wt. % or up to 3 wt. % dispersant.

### Anti-wear Agents

The lubricating composition optionally further includes at least one antiwear agent. Examples of suitable antiwear agents suitable for use herein include titanium compounds, tartrates, tartrimides, oil soluble amine salts of phosphorus compounds, sulfurized olefins, metal dihydrocarbyldithiophosphates (such as zinc dialkyldithiophosphates), phosphites (such as dibutyl phosphite), phosphonates, thiocarbamate-containing compounds, such as thiocarbamate esters, thiocarbamate amides, thiocarbamic ethers, alkylene-coupled thiocarbamates, and bis(S-alkyldithiocarbamyl) disulfides. The antiwear agent may in one embodiment include a tartrate, or tartrimide as described in U.S. Pub. Nos. 2006/0079413; 2006/0183647; and 2010/0081592. The tartrate or tartrimide may contain alkyl-ester groups, where the sum of carbon atoms on the alkyl groups is at least 8. The antiwear agent may, in one embodiment, include a citrate as is disclosed in US Pub. No. 20050198894.

The lubricating composition may in one embodiment further include a phosphorus-containing antiwear agent. Example phosphorus-containing antiwear agents include zinc dialkyldithiophosphates, phosphites, phosphates, phosphonates, and ammonium phosphate salts, and mixtures thereof.

When present, the lubricating composition may include at least 0.01 wt. %, or at least 0.1 wt. %, or at least 0.5 wt. % antiwear agent, and in some embodiments, up to 3 wt. %, or up to 1.5 wt. %, or up to 0.9 wt. antiwear agent.

### Oil-soluble Titanium Compounds

The lubricating composition may include one or more oil-soluble titanium compounds, which may function as antiwear agents, friction modifiers, antioxidants, deposit control additives, or more than one of these functions. Example oil-soluble titanium compounds are disclosed in U.S. Pat. No. 7,727,943 and U.S. Pub. No. 2006/0014651. Example oil soluble titanium compounds include titanium (IV) alkoxides, such as titanium (IV) isopropoxide and titanium (IV) 2 ethylhexoxide. Such alkoxides may be formed from a monohydric alcohol, a vicinal 1,2-diol, a polyol, or mixture thereof. The monohydric alkoxides may have 2 to 16, or 3 to 10 carbon atoms. In one embodiment, the titanium compound comprises the alkoxide of a vicinal 1,2-diol or polyol. 1,2-vicinal diols include fatty acid mono-esters of glycerol, where the fatty acid may be, for example, oleic acid. Other example oil soluble titanium compounds include titanium carboxylates, such as titanium neodecanoate.

When present in the lubricating composition, the amount of oil-soluble titanium compounds is included as part of the antiwear agent.

### Extreme Pressure (EP) agents

The lubricating composition may include an extreme pressure agent. Example extreme pressure agents that are soluble in the oil include sulfur- and chlorosulfur-containing EP agents, dimercaptothiadiazole or CS₂ derivatives of dispersants (typically succinimide dispersants), derivative of chlorinated hydrocarbon EP agents and phosphorus EP agents. Examples of such EP agents include chlorinated wax; sulfurized olefins (such as sulfurized isobutylene), hydrocarbyl-substituted 2,5-dimercapto-1,3,4-thiadiazoles and oligomers thereof, organic sulfides and polysulfides, such as dibenzyldisulfide, bis-(chlorobenzyl) disulfide, dibutyl tetrasulfide, sulfurized methyl ester of oleic acid, sulfurized alkylphenol, sulfurized dipentene, sulfurized terpene, and sulfurized Diels-Alder adducts; phosphosulfurized hydrocarbons such as the reaction product of phosphorus sulfide with turpentine or methyl oleate; phosphorus esters, such as dihydrocarbon and trihydrocarbon phosphites, e.g., dibutyl phosphite, diheptyl phosphite, dicyclohexyl phosphite, pentylphenyl phosphite; dipentylphenyl phosphite, tridecyl phosphite, distearyl phosphite and polypropylene substituted phenol phosphite; metal thiocarbamates, such as zinc dioctyldithiocarbamate and barium heptylphenol diacid; amine salts of alkyl and dialkylphosphoric acids or derivatives including, for example, the amine salt of a reaction product of a dialkyldithiophosphoric acid with propylene oxide and subsequently followed by a further reaction with P₂O₅; and mixtures thereof. Some useful extreme pressure agents are described in US Pat. No. 3,197,405.

When present, the lubricating composition may include at least 0.01 wt. %, or at least 0.1 wt. %, or at least 0.5 wt. % extreme pressure agent, and in some embodiments, up to 3 wt. %, or up to 1.5 wt. %, or up to 0.9 wt. % of the extreme pressure agent.

### Foam Inhibitors

The lubricating composition may include a foam inhibitor. Foam inhibitors that may be useful in the lubricant composition include polysiloxanes; copolymers of ethyl acrylate and 2-ethylhexylacrylate and optionally vinyl acetate; demulsifiers including fluorinated polysiloxanes, trialkyl phosphates, polyethylene glycols, polyethylene oxides, polypropylene oxides and (ethylene oxide-propylene oxide) polymers.

### Viscosity Modifiers

The lubricating composition may include a viscosity modifier. Viscosity modifiers (also sometimes referred to as viscosity index improvers or viscosity improvers) useful in the lubricant composition are usually polymers, including polyisobutylenes, polymethacrylates (PMA) and polymethacrylic acid esters, diene polymers, polyalkylstyrenes, esterified styrene-maleic anhydride copolymers, hydrogenated alkenylarene-conjugated diene copolymers and polyolefins also referred to as olefin copolymer or OCP. PMA's are prepared from mixtures of methacrylate monomers having different alkyl groups. The alkyl groups may be either straight chain or branched chain groups containing from 1 to 18 carbon atoms. Most PMA's are viscosity modifiers as well as pour point depressants. In one embodiment, the viscosity modifier is a polyolefin comprising ethylene and one or more higher olefin, such as propylene.

When present, the lubricating composition may include at least 0.01 wt. %, or at least 0.1 wt. %, or at least 0.3 wt. %, or at least 0.5 wt. % polymeric viscosity modifiers, and in some embodiments, up to 10 wt. %, or up to 5 wt. %, or up to 2.5 wt. % polymeric viscosity modifiers.

### Corrosion Inhibitors and Metal Deactivators

The lubricating composition may include a corrosion inhibitor. Corrosion inhibitors/metal deactivators that may be useful in the exemplary lubricating composition include fatty amines, octylamine octanoate, condensation products of dodecenyl succinic acid or anhydride, and a fatty acid such as oleic acid with a polyamine, derivatives of benzotriazoles (e.g., tolyltriazole), 1,2,4-triazoles, benzimidazoles, 2-alkyldithiobenzimidazoles and 2-alkyldithiobenzothiazoles.

### Pour Point Depressants

The lubricating composition may include a pour point depressant. Pour point depressants that may be useful in the exemplary lubricating composition include polyalphaolefins, esters of maleic anhydride-styrene copolymers, polymethacrylates, polyacrylates, and polyacrylamides.

### Friction Modifiers

The lubricating composition may include a friction modifier. Friction modifiers that may be useful in the exemplary lubricating composition include fatty acid derivatives such as amines, esters, epoxides, fatty imidazolines, condensation products of carboxylic acids and polyalkylene-polyamines and amine salts of alkylphosphoric acids. The friction modifier may be an ash-free friction modifier. Such friction modifiers are those which typically not produce any sulfated ash when subjected to the conditions of ASTM D 874. An additive is referred to as "non-metal containing" if it does not contribute metal content to the lubricant composition. As used herein the term "fatty alkyl" or "fatty" in relation to friction modifiers means a carbon chain having 8 to 30 carbon atoms, typically a straight carbon chain.

In one embodiment, the ash-free friction modifier may be represented by the formula: 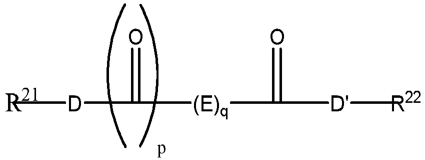 where, D and D' are independently selected from -O-, >NH, >NR²³, an imide group formed by taking together both D and D' groups and forming a R²¹-N< group between two >C=O groups; E is selected from -R²⁴-O-R²⁵-, >CH₂, >CHR²⁶, >CR²⁶R²⁷, >C(OH)(CO₂R²²), >C(CO₂R²²)₂, and >CHOR²⁸; where R²⁴ and R²⁵ are independently selected from >CH₂, >CHR²⁶, >CR²⁶R²⁷, >C(OH)(CO₂R²²), and >CHOR²⁸; q is 0 to 10, with the proviso that when q=1, E is not >CH₂, and when n=2, both Es are not >CH₂; p is 0 or 1; R²¹ is independently hydrogen or a hydrocarbyl group, typically containing 1 to 150 carbon atoms, with the proviso that when R²¹ is hydrogen, p is 0, and q is more than or equal to 1; R²² is a hydrocarbyl group, typically containing 1 to 150 carbon atoms; R²³, R²⁴, R²⁵, R²⁶ and R²⁷ are independently hydrocarbyl groups; and R²⁸ is hydrogen or a hydrocarbyl group, typically containing 1 to 150 carbon atoms, or 4 to 32 carbon atoms, or 8 to 24 carbon atoms. In certain embodiments, the hydrocarbyl groups R²³, R²⁴, and R²⁵, may be linear or predominantly linear alkyl groups.

In certain embodiments, the ash-free friction modifier is a fatty ester, amide, or imide of various hydroxy-carboxylic acids, such as tartaric acid, malic acid lactic acid, glycolic acid, and mandelic acid. Examples of suitable materials include tartaric acid di(2-ethylhexyl) ester (i.e., di(2-ethylhexyl)tartrate), di(C₈-C₁₀)tartrate, di(C₁₂-₁₅)tartrate, di-oleyltartrate, oleyltartrimide, and oleyl maleimide.

In certain embodiments, the ash-free friction modifier may be chosen from long chain fatty acid derivatives of amines, fatty esters, or fatty epoxides; fatty imidazolines such as condensation products of carboxylic acids and polyalkylene-polyamines; amine salts of alkylphosphoric acids; fatty alkyl tartrates; fatty alkyl tartrimides; fatty alkyl tartramides; fatty phosphonates; fatty phosphites; borated phospholipids, borated fatty epoxides; glycerol esters; borated glycerol esters; fatty amines; alkoxylated fatty amines; borated alkoxylated fatty amines; hydroxyl and polyhydroxy fatty amines including tertiary hydroxy fatty amines; hydroxy alkyl amides; metal salts of fatty acids; metal salts of alkyl salicylates; fatty oxazolines; fatty ethoxylated alcohols; condensation products of carboxylic acids and polyalkylene polyamines; or reaction products from fatty carboxylic acids with guanidine, aminoguanidine, urea, or thiourea and salts thereof.

Friction modifiers may also encompass materials such as sulfurized fatty compounds and olefins, sunflower oil or soybean oil monoester of a polyol and an aliphatic carboxylic acid.

In another embodiment the friction modifier may be a long chain fatty acid ester. In another embodiment the long chain fatty acid ester may be a monoester and in another embodiment the long chain fatty acid ester may be a triglyceride.

The amount of the ash-free friction modifier in a lubricant may be 0.1 to 3 percent by weight (or 0.12 to 1.2 or 0.15 to 0.8 percent by weight). The material may also be present in a concentrate, alone or with other additives and with a lesser amount of oil. In a concentrate, the amount of material may be two to ten times the above concentration amounts.

Molybdenum compounds are also known as friction modifiers. The exemplary molybdenum compound does not contain dithiocarbamate moieties or ligands.

Nitrogen-containing molybdenum materials include molybdenum-amine compounds, as described in U.S. Pat. No. 6,329,327, and organomolybdenum compounds made from the reaction of a molybdenum source, fatty oil, and a diamine as described in U.S. Pat. No. 6,914,037. Other molybdenum compounds are disclosed in U.S. Pub. No. 20080280795. Molybdenum amine compounds may be obtained by reacting a compound containing a hexavalent molybdenum atom with a primary, secondary or tertiary amine represented by the formula NR²⁹R³⁰R³¹, where each of R²⁹, R³⁰ and R³¹ is independently hydrogen or a hydrocarbyl group of 1 to 32 carbon atoms and wherein at least one of R²⁹, R³⁰ and R³¹ is a hydrocarbyl group of 4 or more carbon atoms or represented by the formula: 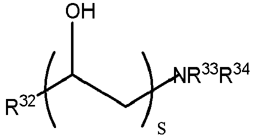 where R³² represents a chain hydrocarbyl group having 10 or more carbon atoms, s is 0 or 1, R³³ and/or R³⁴ represents a hydrogen atom, a hydrocarbyl group, an alkanol group or an alkyl amino group having 2 to 4 carbon atoms, and when s = 0, both R³³ and R³⁴ are not hydrogen atoms or hydrocarbon groups.

Specific examples of suitable amines include monoalkyl (or alkenyl) amines such as tetradecylamine, stearylamine, oleylamine, beef tallow alkylamine, hardened beef tallow alkylamine, and soybean oil alkylamine; dialkyl(or alkenyl)amines such as N-tetradecylmethylamine, N-pentadecylmethylamine, N-hexadecylmethylamine, N-stearylmethylamine, N-oleylmethylamine, N-dococylmethylamine, N-beef tallow alkyl methylamine, N-hardened beef tallow alkyl methylamine, N-soybean oil alkyl methylamine, ditetradecylamine, dipentadecylamine, dihexadecylamine, distearylamine, dioleylamine, didococylamine, bis(2-hexyldecyl)amine, bis(2-octyldodecyl)amine, bis(2-decyltetradecyl)amine, beef tallow dialkylamine, hardened beef tallow dialkylamine, and soybean oil dialkylamine; and trialk(en)ylamines such as tetradecyldimethylamine, hexadecyldimethylamine, octadecyldimethylamine, beef tallow alkyldimethylamine, hardened beef tallow alkyldimethylamine, soybean oil alkyldimethylamine, dioleylmethylamine, tritetradecylamine, tristearylamine, and trioleylamine. Suitable secondary amines have two alkyl (or alkenyl) groups with 14 to 18 carbon atoms.

Examples of the compound containing the hexavalent molybdenum atom include molybdenum trioxides or hydrates thereof (MoO₃·nH₂O), molybdenum acid (H₂MoO₄), alkali metal molybdates (Q₂MoO₄) wherein Q represents an alkali metal such as sodium and potassium, ammonium molybdates {(NH₄)₂MoO₄ or heptamolybdate (NH₄)₆[Mo₇O₂₄].4H₂O}, MoOCl₄, MoO₂Cl₂, MoO₂Br₂, Mo₂O₃Cl₆ and the like. Molybdenum trioxides or hydrates thereof, molybdenum acid, alkali metal molybdates and ammonium molybdates are often suitable because of their availability. In one embodiment, the lubricating composition comprises molybdenum amine compound.

Other organomolybdenum compounds of the invention may be the reaction products of fatty oils, mono-alkylated alkylene diamines and a molybdenum source. Materials of this sort are generally made in two steps, a first step involving the preparation of an aminoamide/glyceride mixture at high temperature, and a second step involving incorporation of the molybdenum.

Examples of fatty oils that may be used include cottonseed oil, groundnut oil, coconut oil, linseed oil, palm kernel oil, olive oil, corn oil, palm oil, castor oil, rapeseed oil (low or high erucic acids), soyabean oil, sunflower oil, herring oil, sardine oil, and tallow. These fatty oils are generally known as glyceryl esters of fatty acids, triacylglycerols or triglycerides.

Examples of some mono-alkylated alkylene diamines that may be used include methylaminopropylamine, methylaminoethylamine, butylaminopropylamine, butylamino-ethylamine, octylaminopropylamine, octylaminoethylamine, dodecylaaminopropylaamine, dodecylaminoethylamine, hexadecylaminopropylamine, hexadecylaminoethylamine, octadecyl-aminopropylamine, octadecylaminoethylamine, isopropyloxypropyl-1,3-diaminopropane, and octyloxypropyl-1,3-diaminopropane. Mono-alkylated alkylene diamines derived from fatty acids may also be used. Examples include N-coco alkyl-1,3-propanediamine (Duomeen^{®}°C), N-tall oil alkyl-1,3-propanediamine (Duomeen^{®}T) and N-oleyl-1,3-propanediamine (Duomeen^{®}O), all commercially available from Akzo Nobel.

Sources of molybdenum for incorporation into the fatty oil/diamine complex are generally oxygen-containing molybdenum compounds include, similar to those above, ammonium molybdates, sodium molybdate, molybdenum oxides and mixtures thereof. One suitable molybdenum source comprises molybdenum trioxide (MoOs).

Nitrogen-containing molybdenum compounds which are commercially available include, for example, Sakuralube^{®} 710 available from Adeka which is a molybdenum amine compound, and Molyvan^{®} 855, available from R.T. Vanderbilt.

The nitrogen-containing molybdenum compound may be present in the lubricant composition at 0.005 to 2 wt. % of the composition, or 0.01 to 1.3 wt. %, or 0.02 to 1.0 wt. % of the composition. The molybdenum compound may provide the lubricant composition with 0 to 1000 ppm, or 5 to 1000 ppm, or 10 to 750 ppm 5 ppm to 300 ppm, or 20 ppm to 250 ppm of molybdenum.

### Demulsifiers

Demulsifiers useful herein include trialkyl phosphates, and various polymers and copolymers of ethylene glycol, ethylene oxide, propylene oxide, and mixtures thereof.

### Seal Swell Agents

Seal swell agents useful herein include sulfolene derivatives such as Exxon Necton-37^{™} (FN 1380) and Exxon Mineral Seal Oil^{™} (FN 3200).

### Example Lubricating Composition

An engine lubricant in different embodiments may have a composition as illustrated in Table 1. All additives are expressed on an oil-free basis.

**TABLE 1: Example Lubricating Compositions**

| Additive | Embodiments (wt. %) | | |
|---|---|---|---|
| | A | B | C |
| Polyisobutylene-substituted phenol or derivative thereof | 0.1 to 20 | 0.5 to 10 | 1 to 5 |
| Overbased Sulfonate Detergent | 0 to 9 | 0.3 to 8 | 1 to 5 |
| Phenol-based detergent | 0 to 10 | 0.5 to 7 | 0.75 to 5 |
| (Borated) Dispersant | 0 to 12 | 0.5 to 8 | 1 to 5 |
| Antioxidant | 0 to 13 | 0.1 to 10 | 0.5 to 5 |
| Antiwear Agent | 0 to 15 | 0.1 to 10 | 0.3 to 5 |
| Corrosion Inhibitor | 0 to 2 | 0.1 to 1 | 0.2 to 0.5 |
| Friction Modifier | 0 to 6 | 0.05 to 4 | 0.1 to 2 |
| Viscosity Modifier | 0 to 10 | 0.5 to 8 | 1 to 6 |
| Other Performance Additives | 0 to 10 | 0 to 8 | 0 to 6 |
| Oil of Lubricating Viscosity | Balance to 100 % | | |

### EXAMPLES

### Example 1: Preparation of Isobutylene-substituted phenol:

Phenol is alkylated with a 350 Mn high vinylidene polyisobutylene (obtained from Texas Petroleum Products) under standard conditions using boron trifluoride as a catalyst, as shown in reaction scheme 1 above.

Phenol (271.5 g) and toluene (131.2 g) are first mixed in a nitrogen blanketed vessel followed by the dropwise addition of BF₃ phenol complex (43.7g) over 20 mins. To this mixture, a solution of 350Mw polyisobutylene (404.3 g, Texas Petroleum Products) diluted in toluene (132.3 g) is added dropwise over 4hrs. The addition rate is controlled to maintain a temperature between 13-21 °C and never exceeded 28°C.

The reaction mixture is then neutralized slowly with calcium hydroxide (84.0 g) with the temperature being held below 28°C. The neutralized reaction mixture is held for 3hrs following which diatomaceous earth (100 g) is charged to the reaction and the neutralization completed with ammonia hydroxide (10 g). The reaction mixture is filtered and the filtrate heated under vacuum to a temperature of 185°C to remove volatiles by distillation. The resulting material is a 350 Mn polyisobutylene-alkylated phenol.

NMR analysis shows the product to be predominately the para-substituted isomer with <3.0% free polyisobutylene present.

The polyisobutylene substituted phenol described in Example 1 was tested for Acute Ecotox Value for Invertebrate using the OECD Guidelines for the Testing of Chemicals, Test No. 202 Daphnia sp. Acute Immobilisation Test (2004). Samples of alkyl-phenols having C4 and C8 alkyl groups were compared to the Sample of Example 1. A published test value for PDDP using the same test method is also included as a comparison.

| **Example** | **Substance ID** | **Acute Ecotox Value for Invertebrate daphnid (EC50)** |
|---|---|---|
| Comparative Ex. 1 | C4 Alkyl Phenol | 4.7 mg/L |
| Comparative Ex. 2 | C8 Alkyl Phenol | 0.09 mg/L |
| Comparative Ex. 3 | PDDP | 0.037 mg/L* |
| Example 1 | 350 Mn Alkyl Phenol | > 100 mg/L |

| | | |
|---|---|---|
| * Sewell & McKenzie (2005a). ACUTE TOXICITY TO DAPHNIA MAGNA. Testing laboratory: Safepharm Laboratories Limited, Shardlow Business Park, Shardlow, Derbyshire, DE72 2GD, UK. Report no.: 1666/027. Owner company: American Chemistry Council, 1300 Wilson Boulevard, Arlington, VA 22209, USA. Report date: 2005-04-26. | | |

An alkyl phenol prepared in accordance with the present invention shows far lower invertebrate toxicity than the comparative materials.

Each of the documents referred to above is incorporated herein by reference. Except in the Examples, or where otherwise explicitly indicated, all numerical quantities in this description specifying amounts of materials, reaction conditions, molecular weights, number of carbon atoms, and the like, are to be understood as modified by the word "about." Unless otherwise indicated, each chemical or composition referred to herein should be interpreted as being a commercial grade material which may contain the isomers, by-products, derivatives, and other such materials which are normally understood to be present in the commercial grade. However, the amount of each chemical component is presented exclusive of any solvent or diluent oil, which may be customarily present in the commercial material, unless otherwise indicated. It is to be understood that the upper and lower amount, range, and ratio limits set forth herein may be independently combined. Similarly, the ranges and amounts for each element of the invention may be used together with ranges or amounts for any of the other elements.

The singular forms "a," "an," and "the" include plural referents unless the context clearly dictates otherwise.

As used herein, the term "hydrocarbyl substituent" or "hydrocarbyl group" is used in its ordinary sense, which is well-known to those skilled in the art. Specifically, it refers to a group having a carbon atom directly attached to the remainder of the molecule and having predominantly hydrocarbon character. By predominantly hydrocarbon character, it is meant that at least 70% or at least 80% of the atoms in the substituent are hydrogen or carbon. Hydrocarbylene groups are the bivalent equivalents of hydrocarbyl groups, i.e., are attached at each end to two parts of the remainder of the molecule.

Examples of hydrocarbyl groups include:
(i) hydrocarbon substituents, that is, aliphatic (e.g., alkyl or alkenyl), alicyclic (e.g., cycloalkyl, cycloalkenyl) substituents, aryl, and aromatic-, aliphatic-, and alicyclic-substituted aromatic substituents, as well as cyclic substituents wherein the ring is completed through another portion of the molecule (e.g., two substituents together form a ring);
(ii) substituted hydrocarbon substituents, that is, substituents containing non-hydrocarbon groups which, in the context of this invention, do not alter the predominantly hydrocarbon nature of the substituent (e.g., halo (especially chloro and fluoro), hydroxy, alkoxy, mercapto, alkylmercapto, nitro, nitroso, and sulfoxy);
(iii) hetero substituents, that is, substituents which, while having a predominantly hydrocarbon character, may contain other than carbon in a ring or chain otherwise composed of carbon atoms.

Representative alkyl groups useful as hydrocarbyl groups may include at least 1, or at least 2, or at least 3, or at least 4 carbon atoms, and in some embodiments, up to 8, or up to 10, or up to 12, or up to 14, or up to 16, or up to 18 carbon atoms. Illustrative examples include methyl, ethyl, propyl, butyl, pentyl, hexyl, heptyl, octyl, 2-ethylhexyl, nonyl, decyl, undecyl, dodecyl, tridecyl, tetradecyl, hexadecyl, stearyl, icosyl, docosyl, tetracosyl, 2-butyloctyl, 2-butyldecyl, 2-hexyloctyl, 2-hexydecyl, 2-octyldecyl, 2-hexyldodecyl, 2-octyldodecyl, 2-decyltetradecyl, 2-dodecylhexadecyl, 2-hexyldecyloctyldecyl, 2-tetradecyloctyldecyl, 4-methyl-2-pentyl, 2-propylheptyl, monomethyl branched-isostearyl, isomers thereof, mixtures thereof, and the like.

Representative alkenyl groups useful as hydrocarbyl groups include C₂-C₁₈ alkenyl groups, such as ethynyl, 2-propenyl, 1-methylene ethyl, 2-butenyl, 3-butenyl, pentenyl, hexenyl, heptenyl, octenyl, 2-ethylhexenyl, nonenyl, decenyl, undecenyl, dodecenyl, tridecenyl, tetradecenyl, hexadecenyl, isomers thereof, mixtures thereof, and the like.

Representative alicyclic groups useful as hydrocarbyl groups include cyclobutyl, cyclopentyl, and cyclohexyl groups.

Representative aryl groups include phenyl, toluyl, xylyl, cumenyl, mesityl, benzyl, phenethyl, styryl, cinnamyl, benzhydryl, trityl, ethylphenyl, propylphenyl, butylphenyl, pentylphenyl, hexylphenyl, heptylphenyl, octylphenyl, nonylphenyl, decylphenyl, undecylphenyl, dodecylphenyl, benzylphenyl, styrenated phenyl, *p*-cumylphenyl, α-naphthyl, β-naphthyl groups, and mixtures thereof.

Representative heteroatoms include sulfur, oxygen, nitrogen, and encompass substituents, such as pyridyl, furyl, thienyl and imidazolyl. In general, no more than two, and in one embodiment, no more than one, non-hydrocarbon substituent will be present for every ten carbon atoms in the hydrocarbyl group. In some embodiments, there are no non-hydrocarbon substituents in the hydrocarbyl group.

Numerical values in the specification and claims of this application should be understood to include numerical values which are the same when reduced to the same number of significant figures and numerical values which differ from the stated value by less than the experimental error of conventional measurement technique of the type described in the present application to determine the value.

As used herein, the term "comprising" is inclusive and does not exclude additional, un-recited elements or method steps. However, in each recitation of "comprising" herein, it is intended that the term also encompasses, as alternative embodiments, the phrases "consisting essentially of" and "consisting of," where "consisting of" excludes any element or steps not specified and "consisting essentially of" permits the inclusion of additional un-recited elements or steps that do not materially affect the basic and novel, and essential characteristics of the composition or method under consideration.

It will be appreciated that variants of the above-disclosed and other features and functions, or alternatives thereof, may be combined into many other different systems or applications. Various presently unforeseen or unanticipated alternatives, modifications, variations or improvements therein may be subsequently made by those skilled in the art which are also intended to be encompassed by the following claims.

Various preferred features and embodiments of the present invention will now be described with reference to the following numbered paragraphs.
1. A polyisobutylene-substituted phenol having the formula: 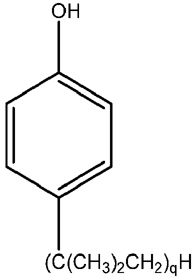 where q is, for example, from 3 to 10, or up to 7, or at least 6, and comprises the reaction product of a phenol with a polyisobutylene having a number average molecular weight of 150 to 500.
2. The polyisobutylene-substituted phenol of paragraph 1, wherein q is one or more of 3, 4, 5, 6, 7, 8, 9, or 10 or mixtures thereof.
3. The polyisobutylene-substituted phenol of paragraph 1 or 2, wherein the polyisobutylene has a molecular weight of 300 to 400.
4. The polyisobutylene-substituted phenol of any of paragraphs 1 to 3, wherein the polyisobutylene is prepared in the absence of a chain transfer agent.
5. The polyisobutylene-substituted phenol of any of paragraphs 1 to 4, wherein the polyisobutylene has a polydispersity of greater than 1.5.
6. A derivative of the polyisobutylene-substituted phenol of paragraph 1, wherein the derivative is selected from phenates, saliginins, Mannich base detergents, oil-soluble zinc salts, polyethers and polyetheramines, methylene coupled hydrocarbyl phenols, and hydrocarbyl substituted amino phenols.
7. A lubricating composition comprising
   (i) an oil of lubricating viscosity and
   (ii) the polyisobutylene-substituted phenol or derivatives thereof of any of paragraphs 1 to 6.
8. The lubricating composition of paragraph 7, wherein the polyisobutylene-substituted phenol is present in amount from 100 to 1000 ppm.
9. The lubricating composition of any of paragraphs 7 or 8, wherein the oil of lubricating viscosity comprises at least one of an API Group I, II, III, IV, and V base oil.
10. The lubricating composition of any one of paragraphs 7 to 9, wherein the oil of lubricating viscosity is at least 10 wt. % of the lubricating composition.
11. The lubricating composition of paragraph 10, wherein the oil of lubricating viscosity is at least 30 wt. % of the lubricating composition.
12. The lubricating composition of any one of paragraphs 7 to 11, wherein the oil of lubricating viscosity is up to 95 wt. % of the lubricating composition.
13. The lubricating composition of any one of paragraphs 7 to 12, further comprising at least one of the group consisting of detergents, antioxidants, dispersants, antiwear agents, friction modifiers, corrosion inhibitors, and combinations thereof.
14. A method of forming a lubricating composition comprising:
   reacting a phenol with a branched polyisobutylene having a number average molecular weight 150 to 500 to form a polyisobutylene-substituted phenol;
   combining the polyolefin-substituted phenol with an oil of lubricating viscosity.
15. The method of paragraph 14, further comprising, preparing a derivative of the polyisobutylene-substituted phenol, wherein the derivative is selected from phenates, saliginins, Mannich base detergents, oil-soluble zinc salts, polyethers and polyetheramines, methylene coupled hydrocarbyl phenols, and hydrocarbyl substituted amino phenols.
16. Use of the lubricating composition of any one of paragraphs 7 to 15 for lubricating a mechanical device.

## Claims

1. A polyisobutylene-substituted phenol having the formula: 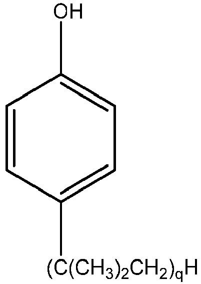 where q is from 3 to 10, or 3 to 7, or at least 6 to 10, and comprises the reaction product of a phenol with a polyisobutylene, with the proviso that the polyisobutylene has a number average molecular weight of 300 to 500.

2. The polyisobutylene-substituted phenol of claim 1, wherein q is one or more of 3, 4, 5, 6, 7, 8, 9, or 10 or mixtures thereof.

3. The polyisobutylene-substituted phenol of claim 1 or 2, wherein the polyisobutylene has a number average molecular weight of 300 to 400.

4. The polyisobutylene-substituted phenol of any of claims 1 to 3, wherein the polyisobutylene is prepared in the absence of a chain transfer agent.

5. The polyisobutylene-substituted phenol of any of claims 1 to 4, wherein the polyisobutylene has a polydispersity of greater than 1.5.

6. A derivative of the polyisobutylene-substituted phenol of any of claims 1 to 5, wherein the derivative is selected from phenates, saliginins, oil-soluble zinc salts, polyethers and polyetheramines, methylene coupled hydrocarbyl phenols, and hydrocarbyl substituted amino phenols.

7. A method of forming a lubricating composition comprising:
combining the derivative of the polyisobutylene-substituted phenol of claim 6 with an oil of lubricating viscosity.

8. Use of the lubricating composition as defined in claim 7 for lubricating a mechanical device.
